(19) Europäisches Patentamt
European Patent Office
Office européen des brevets

(11) EP 4 810 606 A1

(12) **EUROPEAN PATENT APPLICATION**
published in accordance with Art. 153(4) EPC

(43) Date of publication:
**23.09.2026 Bulletin 2026/39**

(21) Application number: **24890760.2**

(22) Date of filing: **14.11.2024**

(51) International Patent Classification (IPC):
***C12N 15/113*** *(2010.01)* ***A61P 19/08*** *(2006.01)*
***A61P 3/00*** *(2006.01)* ***A61K 31/713*** *(2006.01)*
***A61K 47/54*** *(2017.01)* ***A61P 3/06*** *(2006.01)*

(52) Cooperative Patent Classification (CPC):
**A61K 31/713; A61K 47/54; A61P 1/16; A61P 3/00; A61P 3/06; A61P 3/10; A61P 7/00; A61P 9/10; A61P 19/02; A61P 19/08; C12N 15/113**

(86) International application number:
**PCT/CN2024/132007**

(87) International publication number:
**WO 2025/103405 (22.05.2025 Gazette 2025/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.11.2023 CN 202311528615**

(71) Applicant: **BEIJING FOYOU PHARMA CO., LTD**
**Beijing 101113 (CN)**

(72) Inventors:
- **WANG, Shucheng**
  **Beijing 101113 (CN)**
- **HUANG, He**
  **Beijing 101113 (CN)**
- **WANG, Yan**
  **Beijing 101113 (CN)**
- **WANG, Xiaojun**
  **Beijing 101113 (CN)**
- **NIU, Lijiao**
  **Beijing 101113 (CN)**
- **LIN, Guoliang**
  **Beijing 101113 (CN)**
- **CHAN, Yunxia**
  **Beijing 101113 (CN)**
- **GENG, Yuxian**
  **Beijing 101113 (CN)**

(74) Representative: **Engelhard, Markus**
**Boehmert & Boehmert**
**Anwaltspartnerschaft mbB**
**Pettenkoferstrasse 22**
**80336 München (DE)**

# (54) SIRNA MODIFIER AND CONJUGATE FOR INHIBITING ANGPTL3 GENE EXPRESSION, AND USE

(57) The present disclosure relates to a modified siRNA and a conjugate for inhibiting ANGPTL3 gene expression, and use. Specifically, the present disclosure relates to a modified siRNA, a siRNA conjugate and a pharmaceutical composition for inhibiting ANGPTL3 gene expression, use, and a method for inhibiting ANGPTL3 gene expression in cells. The modified siRNA and the conjugate can form an RNA-induced silencing complex (RISC) in the cells, cleaves an mRNA transcribed by an ANGPTL3 gene, specifically inhibits the expression of the ANGPTL3 gene. The modified siRNA and the conjugate are used for treating ANGPTL3 gene-mediated metabolism disorder related diseases, and have important application prospects in clinical disease treatment.

EP 4 810 606 A1

## Description

### TECHNICAL FIELD

The present disclosure belongs to the field of biomedicine. Specifically, the present disclosure relates to a modified siRNA, a siRNA conjugate, a pharmaceutical composition for inhibiting ANGPTL3 gene expression, use thereof, and a method for inhibiting expression of an ANGPTL3 gene in a cell.

### BACKGROUND

Angiopoietin-like protein 3 (ANGPTL3), also known as ANGPL3 or ANG3, is a secreted glycoprotein and is a member of the angiopoietin-like family of secreted factors that regulate lipid metabolism. ANGPTL3 is primarily synthesized in the liver and subsequently secreted into the circulation, eventually being expressed primarily in the liver. ANGPTL3 regulates lipid metabolism by binding to adipose tissue and inhibiting the activity of lipoprotein lipase, specifically by inhibiting the catalytic activity of lipoprotein lipase and endothelial lipase. Inhibition of these two types of enzymes can lead to increased plasma levels of triglycerides, high-density lipoprotein (HDL) and phospholipids. Therefore, low expression of ANGPTL3 can alleviate various diseases associated with dyslipidemia, including chylomicronemia syndrome, type 2 diabetes, familial partial lipodystrophy, hypercholesterolemia, familial hypercholesterolemia, hypertriglyceridemia, non-alcoholic fatty liver, atherosclerosis, knee injury and osteoarthritis, dyslipidemia, and related metabolic diseases.

Dyslipidemia, also known as hyperlipidemia, is a systemic disease characterized by abnormal lipid metabolism or transport that results in plasma lipids exceeding normal values; lipid metabolism disorders can lead to elevated levels of serum lipids, such as triglycerides and/or cholesterol. Elevated serum lipids are strongly associated with hypertension, cardiovascular disease, diabetes, and other pathological conditions. Hypertriglyceridemia is an example of a disorder of lipid metabolism characterized by high blood levels of triglycerides, which is no longer rare and is posing a serious threat to the health of patients worldwide. Current treatments for lipid metabolism disorders include diet, exercise, or the use of statins and other pharmacological therapies, but these do not meet the health needs of most patients. Therefore, the development of novel ANGPTL3 gene expression inhibitors is an important problem that urgently needs to be addressed at present.

### SUMMARY

#### Problems to Be Solved by the Present Disclosure

In view of the problems in the prior art, for example, there is a need to develop more ANGPTL3 inhibitors for treating ANGPTL3-related diseases including abnormal lipid metabolism. The present disclosure aims to provide a series of modified siRNAs, siRNA conjugates, and pharmaceutical compositions for inhibiting ANGPTL3 gene expression, which can inhibit ANGPTL3 gene expression and have important application prospects in the treatment of clinical diseases.

#### Solutions for Solving Problems

[1]. A modified siRNA for inhibiting ANGPTL3 gene expression, comprising a sense strand and an antisense strand, wherein each nucleotide in the modified siRNA is independently a modified or unmodified nucleotide; the sense strand comprises a nucleotide sequence I, the antisense strand comprises a nucleotide sequence II, and the nucleotide sequence I is at least partially reversely complementary to the nucleotide sequence II to form a double-stranded region, wherein the nucleotide sequence I and the nucleotide sequence II are selected from the following sequences:

(1) nucleotide sequence I comprising the nucleotide sequence as shown in SEQ ID NO: 35, and nucleotide sequence II comprising the nucleotide sequence as shown in SEQ ID NO: 36:

5'-GGAUUAUCUUGGAAGUCUA-3' (SEQ ID NO: 35)
5'-UAGACUUCCAAGAUAAUCC-3' (SEQ ID NO: 36);

(2) nucleotide sequence I comprising the nucleotide sequence as shown in SEQ ID NO: 37, and nucleotide sequence II comprising the nucleotide sequence as shown in SEQ ID NO: 38:

5'-GUCUCAAAAUGGAAGGUU$Z_1$-3' (SEQ ID NO: 37)
5'-$Z_2$AACCUUCCAUUUUGAGAC-3' (SEQ ID NO: 38)
wherein $Z_1$ is A, and $Z_2$ is U; or $Z_1$ is U, and $Z_2$ is A;

(3) nucleotide sequence I comprising the nucleotide sequence as shown in SEQ ID NO: 27, and nucleotide sequence II comprising the nucleotide sequence as shown in SEQ ID NO: 28:

5'-GGAAGGUUAUACUCUAUA$Z_3$-3' (SEQ ID NO: 27)
5'-$Z_4$UAUAGAGUAUAACCUUCC-3' (SEQ ID NO: 28)
wherein $Z_3$ is A, and $Z_4$ is U; or $Z_3$ is U, and $Z_4$ is A.

[2]. The modified siRNA according to [1], wherein the nucleotide sequence I is substantially reversely complementary, virtually reversely complementary, or completely reversely complementary to the nucleotide sequence II**;** the substantially reversely complementary means that there are no more than 3 base mismatches between the two nucleotide sequences; the virtually reversely complementary means that there is no more than 1 base mismatch between the two nucleotide sequences; the completely reversely complementary means that there is no base mismatch between the two nucleotide sequences.

[3]. The modified siRNA according to [1] or [2], wherein the sense strand further comprises a nucleotide sequence III, and the antisense strand further comprises a nucleotide sequence IV, wherein the nucleotide sequence III and the nucleotide sequence IV have a length of 0-3 nucleotides; the nucleotide sequence III is linked to the 3' end of the sense strand to form a 3' overhang of the sense strand, and/or the nucleotide sequence IV is linked to the 3' end of the antisense strand to form a 3' overhang of the antisense strand; preferably, the nucleotide sequence III or the nucleotide sequence IV has a length of 0-2 nucleotides; more preferably, the nucleotide sequence III or the nucleotide sequence IV is two thymine deoxyribonucleotides.

[4]. The modified siRNA according to any one of [1]-[3], wherein the double-stranded region has a length of 15-30 nucleotide pairs; preferably, the double-stranded region has a length of 17-23 nucleotide pairs; more preferably, the double-stranded region has a length of 19-23 nucleotide pairs.

[5]. The modified siRNA according to any one of [1]-[4], wherein the sense strand or the antisense strand has 15-30 nucleotides; preferably, the sense strand or the antisense strand has 19-25 nucleotides; more preferably, the sense strand or the antisense strand has 19-23 nucleotides.

[6]. The modified siRNA according to any one of [1]-[5], wherein at least one nucleotide in the sense strand or the antisense strand is a modified nucleotide, and/or at least one phosphoester group is a phosphoester group with a modification group; preferably, the phosphoester group with a modification group is a phosphorothioate diester bond formed by substituting one oxygen atom in a phosphodiester bond in the phosphoester group with a sulfur atom; and/or, the siRNA comprises a sense strand without a 3' overhang nucleotide.

[7]. The modified siRNA according to any one of [1]-[6], wherein the 5' end nucleotide of the antisense strand is not linked to a 5' phosphate group or a 5' phosphate-derived group.

[8]. The modified siRNA according to any one of [1]-[7], wherein the modified nucleotide is selected from a 2'-fluoro-modified nucleotide, a 2'-alkoxy-modified nucleotide, a 2'-substituted alkoxy-modified nucleotide, a 2'-alkyl-modified nucleotide, a 2'-substituted alkyl-modified nucleotide, a 2'-deoxynucleotide, a 2'-amino-modified nucleotide, a 2'-substituted amino-modified nucleotide, a nucleotide analog, and a combination of any two or more thereof; preferably, the modified nucleotide is selected from a 2'-fluoro-modified nucleotide, a 2'-methoxy-modified nucleotide, a 2'-O-$CH_2$-$CH_2$-O-$CH_3$-modified nucleotide, a 2'-O-$CH_2$-CH=$CH_2$-modified nucleotide, a 2'-$CH_2$-$CH_2$-CH=$CH_2$-modified nucleotide, a 2'-deoxynucleotide, a nucleotide analog, and a combination of any two or more thereof; the nucleotide analog is selected from an isonucleotide, LNA, ENA, cET, UNA, and GNA.

[9]. The modified siRNA according to any one of [1]-[8], wherein in the direction from the 5' end to the 3' end, ribonucleotides at positions 7, 9, 10, and 11 in the sense strand are 2'-F-modified ribonucleotides, and ribonucleotides at remaining positions in the sense strand are 2'-O-$CH_3$-modified ribonucleotides.

[10]. The modified siRNA according to any one of [1]-[9], wherein the sense strand comprises phosphorothioate diester bonds located at the positions shown below:

a position between the first nucleotide and the second nucleotide from the 5' end of the sense strand; and
a position between the second nucleotide and the third nucleotide from the 5' end of the sense strand;
or, the sense strand comprises phosphorothioate diester bonds located at the positions shown below:

a position between the first nucleotide and the second nucleotide from the 5' end of the sense strand;
a position between the second nucleotide and the third nucleotide from the 5' end of the sense strand;
a position between the first nucleotide and the second nucleotide from the 3' end of the sense strand; and

a position between the second nucleotide and the third nucleotide from the 3' end of the sense strand.

[11]. The modified siRNA according to any one of [1]-[10], wherein in the direction from the 5' end to the 3' end, ribonucleotides at positions 2, 6, 14, and 16 in the antisense strand are 2'-F-modified ribonucleotides, and ribonucleotides at remaining positions in the antisense strand are 2'-O-$CH_3$-modified ribonucleotides;

or, in the direction from the 5' end to the 3' end, ribonucleotides at positions 2, 6, 8, 9, 14, and 16 in the antisense strand are 2'-F-modified ribonucleotides, and ribonucleotides at remaining positions in the antisense strand are 2'-O-$CH_3$-modified ribonucleotides;
or, in the direction from the 5' end to the 3' end, ribonucleotides at positions 2, 14, and 16 in the antisense strand are 2'-F-modified ribonucleotides, a ribonucleotide at position 6 in the antisense strand is a ribonucleotide modified with a nucleotide derivative GNA, and ribonucleotides at remaining positions in the antisense strand are 2'-O-$CH_3$-modified ribonucleotides;
or, in the direction from the 5' end to the 3' end, ribonucleotides at positions 2, 6, 14, and 16 in the antisense strand are 2'-F-modified ribonucleotides, a ribonucleotide at position 7 in the antisense strand is a ribonucleotide modified with a nucleotide derivative GNA, and the ribonucleotides at remaining positions in the antisense strand are 2'-O-$CH_3$-modified ribonucleotides.

[12]. The modified siRNA according to any one of [1]-[11], wherein the antisense strand comprises phosphorothioate diester bonds located at the positions shown below:

a position between the first nucleotide and the second nucleotide from the 5' end of the antisense strand;
a position between the second nucleotide and the third nucleotide from the 5' end of the antisense strand;
a position between the first nucleotide and the second nucleotide from the 3' end of the antisense strand; and
a position between the second nucleotide and the third nucleotide from the 3' end of the antisense strand.

[13]. The modified siRNA according to any one of [1]-[12], wherein the modified siRNA comprises or is selected from the modified siRNAs in Table 1; preferably, the modified siRNA is N-ER-FY013062M6 or N-ER-FY013062M6D2.
[14]. A siRNA conjugate, comprising the modified siRNA according to any one of [1]-[13], and a conjugated group conjugated to the modified siRNA.
[15].The siRNA conjugate according to [14], wherein the conjugated group has the structure shown below:

formula (I)

formula (III)

formula (IV)

formula (V)

formula (VI)

formula (VII)

formula (VIII)

formula (IX).

[16]. The siRNA conjugate according to [14] or [15], wherein the conjugated group is linked to the 3' end of the sense strand.

[17]. The siRNA conjugate according to [16], wherein the conjugated group is conjugated to the 3' end of the sense strand via a phosphodiester bond;

preferably, the sense strand and the antisense strand of the siRNA conjugate are complementary to form a double-stranded region of the siRNA conjugate, the 3' end of the sense strand forms a blunt end, and the 3' end of the antisense strand has 1-2 overhanging nucleotides extending out of the double-stranded region.

[18]. The siRNA conjugate according to any one of [14]-[17], wherein the siRNA conjugate has the structure shown below:

(formula II),

wherein the double helical structure is a modified siRNA.

[19]. The siRNA conjugate according to any one of [14]-[18], wherein the siRNA conjugate is formed by linking any one of the modified siRNAs shown in Table 1 to a conjugated group;

preferably, in the siRNA conjugate, the sense strand and the antisense strand are selected from the combinations of the sense strand and the antisense strand of the siRNA conjugates shown in Table 2; preferably, the siRNA conjugate is N-ER-FY013062M6L96.

[20]. A pharmaceutical composition, comprising at least one of the following: the modified siRNA according to any one of [1]-[13], and the siRNA conjugate according to any one of [14]-[19].

[21]. The pharmaceutical composition according to [20], wherein the pharmaceutical composition further comprises

one or more pharmaceutically acceptable carriers.

[22]. Use of the modified siRNA according to any one of [1]-[13], the siRNA conjugate according to any one of [14]-[19], or the pharmaceutical composition according to [20] or [21] in at least one of the following:

(1) inhibiting ANGPTL3 gene expression, or preparing a medicament for inhibiting ANGPTL3 gene expression;
(2) preventing or treating a disease associated with abnormal ANGPTL3 gene expression, or preparing a medicament for preventing or treating a disease associated with abnormal ANGPTL3 gene expression;
(3) treating a subject suffering from a disease that would benefit from a reduction in ANGPTL3 gene expression, or preparing a medicament for treating a subject suffering from a disease that would benefit from a reduction in ANGPTL3 gene expression.

[23]. The use according to [22], wherein the disease associated with abnormal ANGPTL3 gene expression is selected from a disease associated with lipid metabolism;
optionally, the disease associated with lipid metabolism is selected from the group consisting of the following diseases:
chylomicronemia syndrome, type 2 diabetes, familial partial lipodystrophy, hypercholesterolemia, familial hypercholesterolemia, non-alcoholic fatty liver, atherosclerosis, hypertriglyceridemia, knee injury and osteoarthritis, dyslipidemia (including mixed dyslipidemia), and fatty liver.

[24]. A method for inhibiting intracellular ANGPTL3 gene expression *in vivo* or *in vitro*, comprising contacting a cell expressing ANGPTL3 with, or administering to a subject in need thereof, a therapeutically effective amount of the modified siRNA according to any one of [1]-[13], the siRNA conjugate according to any one of [14]-[19], or the pharmaceutical composition according to [20] or [21].

[25]. A method for preventing or treating a disease associated with abnormal ANGPTL3 gene expression, comprising administering to a subject in need thereof a therapeutically effective amount of the modified siRNA according to any one of [1]-[13], the siRNA conjugate according to any one of [14]-[19], or the pharmaceutical composition according to [20] or [21],

wherein preferably, the disease associated with abnormal ANGPTL3 gene expression is selected from a disease associated with lipid metabolism;
optionally, the disease associated with lipid metabolism is selected from the group consisting of the following diseases:
chylomicronemia syndrome, type 2 diabetes, familial partial lipodystrophy, hypercholesterolemia, familial hypercholesterolemia, non-alcoholic fatty liver, atherosclerosis, hypertriglyceridemia, knee injury and osteoarthritis, dyslipidemia (including mixed dyslipidemia), and fatty liver.

[26]. The modified siRNA according to any one of [1]-[13], the siRNA conjugate according to any one of [14]-[19], or the pharmaceutical composition according to [20] or [21] for use in therapy.

## Effects of the Present Disclosure

In some embodiments, the modified siRNA provided in the present disclosure is capable of targeting, binding to, and degrading the transcript mRNA of the ANGPTL3 gene to exert an RNA interference effect and inhibit the protein expression of the ANGPTL3 gene. The modified siRNA is an ANGPTL3 inhibitor with a high inhibition rate and good specificity, can efficiently and specifically inhibit the ANGPTL3 gene expression, and can be used for treating ANGPTL3-related diseases including abnormal lipid metabolism. In addition, the modified siRNA of the present disclosure has high stability and relatively good inhibition activity.

In some embodiments, in the present disclosure, the conjugated group is linked to the modified siRNA to obtain the siRNA conjugate, which can be used for efficient targeted delivery to tissues and cells, reduce the impact of the modified siRNA on non-targeted normal tissues and cells, and improve its safety in clinical disease treatment. While maintaining the inhibition activity and stability of the modified siRNA, the conjugate also possesses organ or tissue targeting capability, which can reduce the impact on other tissues or organs and decrease the amount of modified siRNA molecules required, thereby achieving the objectives of lowering toxicity and reducing costs.

Further, the conjugated group in the present disclosure is a group of the structure represented by formula I (GalNAc). GalNAc can be used for targeted delivery into hepatocytes or tissues and is used for efficiently inhibiting the ANGPTL3 gene expression in the liver. The siRNA conjugate of the present disclosure exhibits relatively high inhibition activity against the ANGPTL3 gene *in vivo* and is able to reduce the ANGPTL3 protein level for a long duration.

The siRNA conjugate of the present disclosure primarily accumulates in the liver, has a long retention time in tissues, and exhibits very good stability.

The siRNA conjugate of the present disclosure has relatively low toxicity and an excellent medication safety window.
The siRNA conjugate of the present disclosure can significantly reduce the expression level of the ANGPTL3 protein and triglyceride content in the cynomolgus monkey plasma in cynomolgus monkeys.

## DETAILED DESCRIPTION

### Definitions

Unless otherwise stated, the terms used in the present disclosure have the following meanings.
In the claims and/or specification of the present disclosure, the word "a" or "an" or "the" may refer to "one", but may also refer to "one or more", "at least one", and "one or more than one".
As used in the claims and specification, the word "comprise", "have", "include", or "contain" is intended to be inclusive or open-ended and does not exclude additional, unreferenced elements or method steps.
Throughout this application, the term "about" means that a value includes the standard deviation of error for the device or method being used to determine the value. The numerical ranges and parameters defining the present disclosure are approximations, and the related numerical values in the specific examples have been presented herein as precisely as possible. However, any numerical value inherently and inevitably contains the standard deviation resulting from the testing method or device described above. Accordingly, unless otherwise explicitly indicated, it should be understood that all ranges, quantities, numerical values, and percentages used herein are modified by the word "about". Herein, "about" generally means that the actual numerical value is within $\pm$10%, $\pm$5%, $\pm$1%, or $\pm$0.5% of a specified value or range.
The terms "ANGPTL3", "ANL3", and "angiopoietin like 3" as used in the context of the present disclosure refer to a well-known gene and polypeptide, which are also known in the art as: ANG-5, FHBL2, and ANGPT5. The ANGPTL3 gene and the ANGPTL3 mRNA sequence are, for example, readily available from: gene databases (GenBank), databases (UniProt), Online Mendelian Inheritance in Man (OMIM), and the like.
The term "ANGPTL3 gene" may be a wild-type ANGPTL3 gene, or an ANGPTL3 gene mutant having a sequence variation. Many sequence variations in the ANGPTL3 gene have been identified and can be found in, for example, NCBIdbSNP and UniProt (see, e.g., ncbi.nlm.nih.gov/snp).
The terms "polypeptide" and "protein" are used interchangeably to refer to a chain of at least two amino acid residues linked to each other by a covalent bond (e.g., a peptide bond), it may be a recombinant polypeptide, a natural polypeptide, or a synthetic polypeptide. The polypeptide may be linear or branched, it may comprise modified amino acids, and it may be interrupted by non-amino acids. The term also includes amino acid polymers that have been modified (e.g., disulfide bond formation, glycosylation, lipidation, acetylation, phosphorylation, or any other manipulation, such as conjugation to a labeling moiety).
The term "target sequence" as used in the context of the present disclosure refers to a contiguous portion of a nucleotide sequence of an mRNA molecule formed during transcription of a target gene, including mRNA that is a product of RNA processing of a primary transcript.
In some embodiments, the target sequence is a nucleotide sequence consisting of no less than 16, 17, 18, 19, 20, 21, 22, 23, 24, 25, 26, 27, 28, 29, 30, 50, 80, 100, 150, 200, 300, 400, 500, 600, or 700 contiguous linked nucleosides. Illustratively, the target sequence is a nucleotide sequence consisting of 19, 20, 21, 22, 23, 24, 25, 26, 27, 28, 29, 30, 31, 32, 33, 34, 35, 38, 53, 54, 65, 125, 69, 97, 80, 128, 164, or 190 contiguous linked nucleosides. In some optional embodiments, the target sequence may comprise another shorter target sequence. In some embodiments, the target sequence may comprise one or more shorter target sequences. It should be understood that two or more shorter target sequences contained within the same target sequence have the same characteristics.
In some embodiments, the target gene is an ANGPTL3 gene. In some embodiments, a target portion of the gene sequence (i.e., the portion corresponding to the target sequence in the mRNA sequence) will be at least sufficiently long to serve as a substrate for iRNA-guided cleavage at or near the portion of the nucleotide sequence of the mRNA molecule formed during transcription of the ANGPTL3 gene.
In the art, "G", "C", "A", "T", and "U" generally represent the bases of guanine, cytosine, adenine, thymine, and uracil, respectively, but it is also generally known in the art that "G", "C", "A", "T", and "U" generally also represent nucleotides comprising guanine, cytosine, adenine, thymine, and uracil as the base, respectively; this is a common way of representing deoxyribonucleic acid sequences and/or ribonucleic acid sequences; therefore, in the context of the present disclosure, the meanings represented by "G", "C", "A", "T", and "U" include the various possible cases described above. However, it should be understood that the term "ribonucleotide" or "nucleotide" may also refer to a modified nucleotide (as further described below) or a nucleotide having an alternative replacement moiety, and "nucleotide" and "nucleic acid nucleotide" in the present disclosure are used interchangeably. Those skilled in the art will recognize that guanine, cytosine, adenine, and uracil may be replaced by other moieties without substantially altering the base pairing properties of

an oligonucleotide (including a nucleotide having such replacement moiety). For example, without limitation, a nucleotide comprising inosine as its base may base-pair with a nucleotide comprising adenine, cytosine, or uracil. Thus, nucleotides comprising uracil, guanine, or adenine may be replaced in the nucleotide sequences of the dsRNA characterized in the present disclosure by a nucleotide comprising, for example, inosine. In another example, adenine and cytosine anywhere in the oligonucleotide can be replaced with guanine and uracil, respectively, to form G-U wobble base pairing with the target mRNA. Sequences comprising such replacement moieties are suitable for use in the compositions and methods characterized in the present disclosure.

In the present application, "5'-nucleotide" refers to a nucleotide in which the phosphate group is linked to the 5' carbon of the pentose sugar, which is the major type of nucleotide that exists freely in organisms. "3'-nucleotide" refers to a nucleotide in which the phosphate group is linked to the 3'-carbon of the pentose sugar. For example, it may include adenosine-3'-phosphate, guanosine-3'-phosphate, cytidine-3'-phosphate, uridine-3'-phosphate, 2'-deoxythymidine-3'-phosphate, 2'-O-methyladenosine-3'-phosphate, 2'-O-methyladenosine-3'-phosphorothioate, 2'-fluoroadenosine-3'-phosphate, 2'-fluoroadenosine-3'-phosphorothioate, 2'-O-methylguanosine-3'-phosphate, 2'-O-methylguanosine-3'-phosphorothioate, 2'-fluoroguanosine-3'-phosphate, 2'-fluoroguanosine-3'-phosphorothioate, 2'-O-methylcytidine-3'-phosphate, 2'-O-methylcytidine-3'-phosphorothioate, 2'-fluorocytidine-3'-phosphate, 2'-fluorocytidine-3'-phosphorothioate, 2'-O-methyluridine-3'-phosphate, 2'-O-methyluridine-3'-phosphorothioate, 2'-fluorouridine-3'-phosphate, 2'-fluorouridine-3'-phosphorothioate, and 2'-deoxythymidine-3'-phosphorothioate. This definition may apply to modified or unmodified nucleoside phosphoramidite monomers.

The terms "iRNA," "RNAi reagent," "iRNA reagent," and "RNA interference agent" as used in the context of the present disclosure are used interchangeably herein and refer to a term as defined herein that includes siRNA and mediates targeted cleavage of an RNA transcript through the RNA-induced silencing complex (RISC) pathway. iRNA directs sequence-specific degradation of mRNA through a process known as RNA interference (RNAi). The iRNA modulates, e.g., inhibits, the expression of a target gene in a cell (e.g., a cell of a subject (e.g., a mammalian subject)).

Generally, the majority of nucleotides of each strand of a dsRNA molecule are ribonucleotides, but as detailed herein, each or both of the two strands may also include one or more non-ribonucleotides, e.g., deoxyribonucleotides and/or modified nucleotides. In addition, as used in the present disclosure, "siRNA" may include ribonucleotides and phosphate backbones with chemical modifications, and the like. These modifications can include all types of modifications disclosed herein or known in the art.

The term "isonucleotide" as used in the context of the present disclosure refers to a compound formed by changing a position of a base on a ribose ring in a nucleotide, for example, a compound formed by connecting a base not to the 1'-position of the ribose ring, but to the 2'-position or 3'-position of the ribose ring.

In some embodiments, the siRNA of the present disclosure interacts with an mRNA sequence transcribed from a target gene (e.g., an mRNA sequence transcribed from the ANGPTL3 gene) to direct the cleavage of the target RNA. Without wishing to be bound by theory, long double stranded RNA introduced into cells is broken down into siRNA by a type III endonuclease known as Dicer (Sharp et al., Genes Dev., 2001, 15:485). Dicer (a ribonuclease III-like enzyme) processes dsRNAs into 19-23 base pair short interfering RNAs with characteristic two-base 3' overhangs (Bernstein et al., (2001) Nature, 409:363). These siRNAs are then incorporated into an RNA-induced silencing complex (RISC), where one or more helicases unwind the siRNA duplex, enabling the complementary antisense strand to guide target recognition (Nykanen et al., (2001) Cell, 107:309). Upon binding to the appropriate target mRNA, one or more endonucleases within the RISC cleave the target to induce silencing (Elbashir et al., (2001) Genes Dev., 15:188).

The term "overhanging nucleotide" as used in the context of the present disclosure refers to one or more unpaired nucleotides that protrude from the duplex structure of dsRNA when one 3' end of one strand of siRNA extends beyond the 5' end of the other strand, or vice versa. "Blunt end" means that there are no unpaired nucleotides at that end of the siRNA, i.e., no nucleotide overhang. "Blunt-ended" siRNA is a dsRNA that is fully double-stranded along its entire length, meaning that there are no nucleotide overhangs at either end of the molecule.

The term "antisense strand" refers to a nucleic acid strand of a region of siRNA that is substantially complementary to a target sequence (e.g., derived from human ANGPTL3 mRNA). In the case where the complementary region is not completely complementary to the target sequence, mismatches are most tolerable in end regions, and if mismatches occur, they are typically within one or more end regions, for example, within 5, 4, 3, 2, or 1 nucleotide(s) of the 5' and/or 3' ends.

The term "sense strand" refers to a nucleic acid strand of siRNA that contains a region substantially complementary to a region of the antisense strand.

The terms "complementary" and "reversely complementary" are used interchangeably and have the meaning well known to those skilled in the art, that is, in a double-stranded nucleic acid molecule, the bases of one strand are paired with the bases of the other strand in a complementary manner. In DNA, the purine base adenine (A) is always paired with the pyrimidine base thymine (T) (or uracil (U) in RNA), and the purine base guanine (G) is always paired with the pyrimidine base cytosine (C). Each base pair comprises a purine and a pyrimidine. When adenines of one strand are always paired with thymines (or uracils) of the other strand and guanines are always paired with cytosines, the two strands are considered

complementary to each other, and the sequence of one strand can be deduced from the sequence of its complementary strand. Accordingly, "mismatch" in the art means that in a double-stranded nucleic acid, the bases in the corresponding positions are not paired in a complementary manner.

The term "substantially reversely complementary" means that there are no more than 3 base mismatches between the two nucleotide sequences involved, i.e., there are 1, 2, or 3 base mismatches between the two nucleotide sequences involved; "completely complementary" means that there is no base mismatch between the two nucleotide sequences.

The terms "complementary," "completely complementary", and "substantially complementary" may be used with respect to the base matching between the sense strand and the antisense strand of dsRNA, or between the antisense strand of dsRNA and a target sequence, as will be understood from the context of their use.

In the text above and below, particularly when the preparation method for the modified siRNA, the conjugate thereof, or the pharmaceutical composition of the present disclosure is described, unless otherwise specified, the nucleoside monomer refers to a modified or unmodified nucleoside phosphoramidite monomer used in phosphoramidite solid-phase synthesis according to the type and order of nucleotides in the siRNA or siRNA conjugate to be prepared. Phosphoramidite solid-phase synthesis is a method used in RNA synthesis well known to those skilled in the art. The nucleoside monomers used in the present disclosure are all commercially available.

The term "inhibit" is used interchangeably with "decrease", "silence", "down-regulate", "suppress", and other similar terms, and includes any level of inhibition.

The term "inhibiting ANGPTL3 gene expression" includes inhibiting the expression of any ANGPTL3 gene (e.g., a mouse ANGPTL3 gene, a rat ANGPTL3 gene, a monkey ANGPTL3 gene, or a human ANGPTL3 gene) and a variant (e.g., a naturally occurring variant) or mutant of an ANGPTL3 gene. Thus, the ANGPTL3 gene may be a wild-type ANGPTL3 gene, a mutant ANGPTL3 gene, or a transgenic ANGPTL3 gene in the case of genetically manipulated cells, cell populations, or organisms.

"Inhibiting ANGPTL3 gene expression" includes any level of inhibition of an ANGPTL3 gene, e.g., at least partial inhibition of expression of an ANGPTL3 gene, such as inhibition of at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99%.

The term "each independently" means that at least two groups (or ring systems) with the same or similar range of values in the structure may have the same or different meanings under a particular circumstance. For example, if substituent X and substituent Y are each independently hydrogen, hydroxyl, alkyl, or aryl, then when substituent X is hydrogen, substituent Y may be hydrogen, or may be hydroxyl, alkyl, or aryl; similarly, when substituent Y is hydrogen, substituent X may be hydrogen, or may be hydroxyl, alkyl, or aryl.

The term "alkyl" includes linear, branched, or cyclic saturated alkyl groups. For example, the alkyl includes, but is not limited to, methyl, ethyl, propyl, cyclopropyl, n-butyl, isobutyl, sec-butyl, tert-butyl, cyclobutyl, n-pentyl, cyclohexyl, and similar groups. Illustratively, "C1-6" in "C1-6 alkyl" refers to a group containing 1, 2, 3, 4, 5, or 6 carbon atoms arranged in a linear, branched, or cyclic form.

The term "alkoxy" as used herein refers to an alkyl group attached to the rest of the molecule via an oxygen atom (-O-alkyl), wherein the alkyl is as defined herein. Non-limiting examples of the alkoxy include methoxy, ethoxy, trifluoromethoxy, difluoromethoxy, n-propoxy, isopropoxy, n-butoxy, tert-butoxy, n-pentoxy, and the like.

The term "treat", "treating", or "treatment" refers to contacting (e.g., administering) the siRNA, the modified siRNA, the siRNA conjugate, or the pharmaceutical composition to a subject after the onset of a disease to alleviate symptoms of the disease as compared to when the subject is not in contact with the siRNA, the modified siRNA, the siRNA conjugate, or the pharmaceutical composition. The term does not mean that the symptoms of the disease must be completely inhibited. "Onset of a disease" refers to the appearance of the symptoms of the disease in the body.

The term "prevent", "preventing", or "prevention" refers to contacting (e.g., administering) the siRNA, the modified siRNA, the siRNA conjugate, or the pharmaceutical composition to a subject before the onset of a disease to alleviate symptoms after the onset of a disease as compared to when the subject is not in contact with the siRNA, the modified siRNA, the siRNA conjugate, or the pharmaceutical composition. The term does not mean that the onset of the disease must be completely inhibited.

The term "effective amount" refers to an amount or dose of the modified siRNA, the siRNA conjugate, or the pharmaceutical composition of the present disclosure which generates expected effects in a patient in need of treatment or prevention after administration to the patient in a single or multiple doses. An effective amount can be readily determined by an attending physician, as a person skilled in the art, by considering a variety of factors as follows: the species of a mammal; its size, age, and general health condition; the specific disease involved; the extent or severity of the disease; the response of the individual patient; the specific antibody administered; the mode of administration; the bioavailability

characteristics of the administered formulation; the selected dosing regimen; and the use of any concomitant therapy.

The term "disease associated with abnormal ANGPTL3 gene expression" is a disease or disorder associated with a metabolic disorder. The term "disease associated with abnormal ANGPTL3 gene expression" includes diseases, disorders, or conditions that would benefit from reduction in ANGPTL3 expression (i.e., "ANGPTL3-related disease"). Such diseases are typically closely related to changes in plasma triglyceride (TG), high-density lipoprotein (HDL), low-density lipoprotein (LDL), and total cholesterol (TC) levels. Non-limiting examples of the disease associated with abnormal ANGPTL3 gene expression include chylomicronemia syndrome, type 2 diabetes, familial partial lipodystrophy, hypercholesterolemia, familial hypercholesterolemia, non-alcoholic fatty liver, atherosclerosis, hypertriglyceridemia, knee injury and osteoarthritis, dyslipidemia and related metabolic diseases, familial hypobetalipoproteinemia, hyperlipidemia, obesity, primary nephrotic syndrome, coronary heart disease, malignancies, and the like (see, e.g., Minicocci et al., Mutations in the ANGPTL3 gene and familial combined hypolipidemia: a clinical and biochemical characterization. J Clin Endocrinol Metab, 2012, 97(7): E1266-1275; Kaplan et al., Regulation of the angiopoietin-like protein 3 gene by LXR. J Lipid Res, 2003, 44(1): 136-143; Cheng Haipeng et al., Lipoprotein lipase degradation and metabolic disorders, Chinese Journal of Arteriosclerosis, 2017, 25(05): 513-518; Rao Jia et al., Expression of ANGPTL3 in children with primary nephrotic syndrome, Chinese Journal of Nephrology, 2006, 22(05): 286-290; Stitziel et al., ANGPTL3 deficiency and protection against coronary artery disease. J Am Coll Cardiol, 2017, 69(16): 2054-2063; Wang PF et al., Clinical significance of angiopoietin-like protein 3 expression in patients with glioblastoma. Neoplasma, 2016, 63(1): 93-98).

The term "pharmaceutically acceptable excipient" or "pharmaceutically acceptable carrier" refers to an auxiliary material widely used in the field of pharmaceutical production. The primary objective of using an excipient is to provide a pharmaceutical composition that is safe to use and stable in properties, and/or has specific functionality, as well as to provide a method for dissolving an active ingredient at a desired rate or promoting effective absorption of an active ingredient in a subject to whom a medicament is administered. The pharmaceutically acceptable excipient may be an inert filler or an efficacy ingredient that provides a certain function for the pharmaceutical composition (e.g., stabilizing the overall pH of the composition or preventing the degradation of the active ingredient in the composition). Non-limiting examples of the pharmaceutically acceptable excipient include, but are not limited to, binders, suspending agents, emulsifiers, diluents (or fillers), granulating agents, adhesives, disintegrants, lubricants, anti-adherents, glidants, wetting agents, gelling agents, absorption delaying agents, dissolution inhibitors, enhancers, adsorbents, buffers, chelating agents, preservatives, colorants, flavoring agents, sweeteners, and the like.

The term "pharmaceutical composition" refers to a mixture consisting of one or more of the modified siRNAs or the conjugates thereof of the present disclosure and a pharmaceutically acceptable excipient/carrier. The pharmaceutical composition is intended to facilitate the administration of the modified siRNA or the conjugate thereof of the present disclosure to an organism.

The pharmaceutical composition in the present disclosure can be prepared using any method known to those skilled in the art, for example, conventional mixing, dissolving, granulating, emulsifying, levigating, encapsulating, entrapping, and/or lyophilizing processes.

In the present disclosure, the route of administration may be varied or adjusted in any applicable way to meet the needs of the nature of the drug, the convenience of patients and medical personnel, and other relevant factors.

The term "individual", "patient", or "subject" as used in the context of the present disclosure includes mammals. The mammals include, but are not limited to, domestic animals (e.g., cattle, goats, cats, dogs, and horses), primates (e.g., human and non-human primates such as monkeys), rabbits, and rodents (e.g., mice and rats).

Unless defined otherwise, or clear from the background, all technical and scientific terms used in the present disclosure have the same meaning as commonly understood by those of ordinary skill in the art to which the present disclosure pertains.

## Modified siRNA

A first aspect of the present disclosure provides a modified siRNA. The modified siRNA can improve stability of siRNA while maintaining relatively high ANGPTL3 mRNA inhibition activity.

The modified siRNA of the present disclosure comprises a sense strand and an antisense strand, wherein the sense strand comprises a nucleotide sequence I, the antisense strand comprises a nucleotide sequence II, and the nucleotide sequence I is at least partially reversely complementary to the nucleotide sequence II to form a double-stranded region, wherein the nucleotide sequence I and the nucleotide sequence II are selected from the following sequences:

(1) nucleotide sequence I comprising the nucleotide sequence as shown in SEQ ID NO: 35, and nucleotide sequence II comprising the nucleotide sequence as shown in SEQ ID NO: 36:

5’-GGAUUAUCUUGGAAGUCUA-3’ (SEQ ID NO: 35)
5’-UAGACUUCCAAGAUAAUCC-3’ (SEQ ID NO: 36);

(2) nucleotide sequence I comprising the nucleotide sequence as shown in SEQ ID NO: 37, and nucleotide sequence II comprising the nucleotide sequence as shown in SEQ ID NO: 38:

5'-GUCUCAAAAUGGAAGGUU$Z_1$-3' (SEQ ID NO: 37)
5'-$Z_2$AACCUUCCAUUUUGAGAC-3' (SEQ ID NO: 38)
wherein $Z_1$ is A, and $Z_2$ is U; or $Z_1$ is U, and $Z_2$ is A;

(3) nucleotide sequence I comprising the nucleotide sequence as shown in SEQ ID NO: 27, and nucleotide sequence II comprising the nucleotide sequence as shown in SEQ ID NO: 28:

5'-GGAAGGUUAUACUCUAUA$Z_3$-3' (SEQ ID NO: 27)
5'-$Z_4$UAUAGAGUAUAACCUUCC-3' (SEQ ID NO: 28)
wherein $Z_3$ is A, and $Z_4$ is U; or $Z_3$ is U, and $Z_4$ is A.

In some embodiments, the nucleotide sequence I is substantially reversely complementary, virtually reversely complementary, or completely reversely complementary to the nucleotide sequence II; the substantially reversely complementary means that there are no more than 3 base mismatches between the two nucleotide sequences; the virtually reversely complementary means that there is no more than 1 base mismatch between the two nucleotide sequences; the completely reversely complementary means that there is no base mismatch between the two nucleotide sequences.

In some embodiments, the sense strand further comprises a nucleotide sequence III, and the antisense strand further comprises a nucleotide sequence IV, wherein the nucleotide sequence III and the nucleotide sequence IV have a length of 0-3 nucleotides; the nucleotide sequence III is linked to the 3' end of the sense strand to form a 3' overhang of the sense strand, and/or the nucleotide sequence IV is linked to the 3' end of the antisense strand to form a 3' overhang of the antisense strand; preferably, the nucleotide sequence III or the nucleotide sequence IV has a length of 0-2 nucleotides; more preferably, the nucleotide sequence III or the nucleotide sequence IV is two thymine deoxyribonucleotides (TT).

In some embodiments, the double-stranded region has a length of 15-30 nucleotide pairs. In other embodiments, the double-stranded region has a length of 17-23 nucleotide pairs. In other embodiments, the double-stranded region has a length of 19-23 nucleotide pairs. In yet other embodiments, the double-stranded region has a length of 19, 21, or 23 nucleotide pairs.

The sense strand and the antisense strand provided in the present disclosure are identical or different in length, and in some embodiments, the sense strand or the antisense strand has 15-30 nucleotides. In other embodiments, the sense strand or the antisense strand has 19-25 nucleotides. In other embodiments, the sense strand or the antisense strand has 19-23 nucleotides. A length ratio of the sense strand to the antisense strand of the siRNA provided in the present disclosure may be 15/15, 16/16, 17/17, 18/18, 19/19, 19/20, 19/21, 19/22, 19/23, 20/19, 20/20, 20/21, 20/22, 20/23, 21/19, 21/20, 21/21, 21/22, 21/23, 22/19, 22/20, 22/21, 22/22, 22/23, 23/19, 23/20, 23/21, 23/22, 23/23, 24/24, 25/25, 26/26, 27/27, 28/28, 29/29, 30/30, 22/24, 22/25, 22/26, 23/24, 23/25, 23/26, or the like. In some embodiments, the length ratio of the sense strand to the antisense strand of the siRNA is 19/19, 21/21, 19/21, or 21/20, and at this time, the siRNA of the present disclosure has better cellular mRNA silencing activity.

In some embodiments, the siRNA of the present disclosure comprises at least 1 modified nucleotide.

Illustratively, a modified nucleotide has the structure shown below:

;

wherein Base represents a base, e.g., A, U, G, C, or T. The hydroxyl at the 2'-position of the ribosyl group is substituted with R. The hydroxyl at the 2'-position of these ribosyl groups may be substituted with various groups known to those skilled in the art, such as a 2'-fluoro (2'-F)-modified nucleotide, a 2'-alkoxy-modified nucleotide, a 2'-substituted alkoxy-modified nucleotide, a 2'-alkyl-modified nucleotide, a 2'-substituted alkyl-modified nucleotide, or a 2'-deoxyribonucleotide.

In some embodiments, the 2'-alkoxy-modified nucleotide is a 2'-methoxy (2'-OMe, 2'-O-$CH_3$)-modified nucleotide or the like.

In some embodiments, the 2'-substituted alkoxy-modified nucleotide is a 2'-methoxyethoxy (2'-O-$CH_2$-$CH_2$-O-$CH_3$)-modified nucleotide, a 2'-O-$CH_2$-CH=$CH_2$-modified nucleotide, or the like.

In some embodiments, the 2'-substituted alkyl-modified nucleotide is a 2'-$CH_2$-$CH_2$-CH=$CH_2$-modified nucleotide or the like.

In some embodiments, the modification of the nucleotide is a modification of the base. The modifications of the base may be of various types known to those skilled in the art. Illustratively, The modifications of the base include, but are not limited to, $m^6A$, Ψ, $m^1A$, $m^5A$, $ms^2i^6A$, $i^6A$, $m^3C$, $m^5C$, $ac^4C$, $m^7G$, $m^{2,2}G$, $m^2G$, $m^1G$, Q, $m^5U$, $mcm^5U$ , $ncm^5U$ , $ncm^5Um$, D, $mcm^5s^2U$, Inosine (I), $hm^5C$, $s^4U$, $s^2U$, azobenzene, Cm, Um, Gm, $t^6A$, yW, $ms^2t^6A$, or a derivative thereof.

In some embodiments, a nucleotide derivative refers to a compound that can replace a nucleotide in a nucleic acid but has a structure different from adenine ribonucleotides, guanine ribonucleotides, cytosine ribonucleotides, uracil ribonucleotides, or thymine deoxyribonucleotides. In some embodiments, the nucleotide derivative may be an isonucleotide, a bridged nucleic acid (BNA for short), or an acyclic nucleotide. BNA refers to a constrained or inaccessible nucleotide. The BNA may contain a five-membered, six-membered, or seven-membered ring bridge structure with a "fixed" C3'-endosugar constriction. Typically, the bridge is incorporated at the 2'- and 4'-positions of the ribose to provide a 2',4'-BNA nucleotide, such as LNA, ENA, and cET.

LNA is represented by formula (1), ENA is represented by formula (2), and cET is represented by formula (3):

formula (1) formula (2) formula (3).

Acyclic nucleotides are a class of nucleotides formed by opening the sugar ring of nucleotides, such as an unlocked nucleic acid (UNA) or a glycerol nucleic acid (GNA), wherein UNA is represented by formula (4), and GNA is represented by formula (5):

formula (4) formula (5).

In formula (4) and formula (5), R is selected from H, OH, and alkoxy (O-alkyl).

In some embodiments, the nucleotide derivative modification refers to replacement of a nucleotide in a nucleic acid with a nucleotide derivative. Illustratively, the nucleotide derivative is selected from an isonucleotide, LNA, ENA, cET, UNA, and GNA.

In some embodiments, the replacement of the nucleotide in the nucleic acid with the isonucleotide is also referred to as an isonucleoside modification in the context of the present disclosure. In some embodiments, the isonucleoside modification comprises incorporating an isonucleoside at one or more sites of the sense strand and/or the antisense strand of the siRNA to be modified to replace a natural nucleoside for coupling at the corresponding position.

In some embodiments, the isonucleoside modification employs a D-isonucleoside modification. In other embodiments, the isonucleoside modification employs an L-isonucleoside modification. In still other embodiments, the isonucleoside modification employs a D-isonucleoside modification and an L-isonucleoside modification.

In some embodiments, the modified siRNA comprises a modification of a phosphodiester bond at least one position. In some embodiments, the modification of the phosphodiester bond means that one oxygen atom in the phosphodiester bond is substituted with a sulfur atom to form a phosphorothioate diester bond. The phosphorothioate diester bond can stabilize the double-stranded structure of the siRNA and maintain the specificity of base pairing. Illustratively, the phosphorothioate diester bond has the structure shown below:

.

In some embodiments, the siRNA comprises a sense strand without a 3' overhang nucleotide; that is, while the sense strand of the siRNA may have a 3' overhang nucleotide, a blunt end is formed by removing the 3' overhang nucleotide from the sense strand. In some embodiments, when the nucleotide sequence of the sense strand and the nucleotide sequence of the antisense strand are complementary to form a double-stranded region, and there is no overhanging nucleotide at the 3' end of the sense strand, the nucleotide sequence III is added to the 3' end of the sense strand as the overhanging nucleotide. Subsequently, after a chemical modification of the nucleotide sequence formed by linking the nucleotide sequence III to the 3' end of the sense strand is completed, the nucleotide sequence III is removed, and correspondingly, the sense strand of the siRNA forms a blunt end.

In some embodiments, in the direction from the 5' end to the 3' end, ribonucleotides at positions 7, 9, 10, and 11 in the sense strand are 2'-F-modified ribonucleotides, and ribonucleotides at remaining positions in the sense strand are 2'-O-$CH_3$-modified ribonucleotides.

In some embodiments, in the direction from the 5' end to the 3' end, the sense strand comprises phosphorothioate diester bonds located at the positions shown below:

a position between the first nucleotide and the second nucleotide from the 5' end of the sense strand; and
a position between the second nucleotide and the third nucleotide from the 5' end of the sense strand;
or, the sense strand comprises phosphorothioate diester bonds located at the positions shown below:

a position between the first nucleotide and the second nucleotide from the 5' end of the sense strand;
a position between the second nucleotide and the third nucleotide from the 5' end of the sense strand;
a position between the first nucleotide and the second nucleotide from the 3' end of the sense strand; and
a position between the second nucleotide and the third nucleotide from the 3' end of the sense strand.

Herein, the 5' end nucleotide of the sense strand is not linked to a 5' phosphate group or a 5' phosphate-derived group (i.e., the ribosyl group of the 5' end nucleotide is a 5' hydroxyl group), and the structure is represented by formula X:

formula (X)

wherein Base represents a base, e.g., A, U, G, C, or T; R is hydroxyl or is substituted with various groups known to those skilled in the art; for example, R may be 2'-fluoro (2'-F), 2'-alkoxy, 2'-substituted alkoxy, 2'-alkyl, 2'-substituted alkyl, 2'-amino, 2'-substituted amino, or a 2'-deoxynucleotide.

Illustratively, a structure of the 5' phosphate group is:

;

a structure of the 5' phosphate-derived group includes, but is not limited to:

(EVP),

and the like.

In some embodiments, in the direction from the 5' end to the 3' end, the antisense strand comprises the following modifications: in the direction from the 5' end to the 3' end, ribonucleotides at positions 2, 6, 14, and 16 in the antisense strand are 2'-F-modified ribonucleotides, and ribonucleotides at remaining positions in the antisense strand are 2'-O-$CH_3$-modified ribonucleotides;

in some embodiments, in the direction from the 5' end to the 3' end, ribonucleotides at positions 2, 6, 8, 9, 14, and 16 in the antisense strand are 2'-F-modified ribonucleotides, and ribonucleotides at remaining positions in the antisense strand are 2'-O-$CH_3$-modified ribonucleotides;
in some embodiments, in the direction from the 5' end to the 3' end, ribonucleotides at positions 2, 14, and 16 in the antisense strand are 2'-F-modified ribonucleotides, a ribonucleotide at position 6 in the antisense strand is a ribonucleotide modified with a nucleotide derivative GNA, and ribonucleotides at remaining positions in the antisense strand are 2'-O-$CH_3$-modified ribonucleotides;
in some embodiments, in the direction from the 5' end to the 3' end, ribonucleotides at positions 2, 6, 14, and 16 in the antisense strand are 2'-F-modified ribonucleotides, a ribonucleotide at position 7 in the antisense strand is a ribonucleotide modified with a nucleotide derivative GNA, and the ribonucleotides at remaining positions in the antisense strand are 2'-O-$CH_3$-modified ribonucleotides.

In some embodiments, in the direction from the 5' end to the 3' end, the antisense strand comprises phosphorothioate diester bonds located at the positions shown below:

a position between the first nucleotide and the second nucleotide from the 5' end of the antisense strand;
a position between the second nucleotide and the third nucleotide from the 5' end of the antisense strand;
a position between the first nucleotide and the second nucleotide from the 3' end of the antisense strand; and
a position between the second nucleotide and the third nucleotide from the 3' end of the antisense strand.

In some embodiments, in the direction from the 5' end to the 3' end, the nucleotide at the 5' end of the antisense strand is not linked to a 5' phosphate group or a 5' phosphate-derived group.

Herein, when the 5' end nucleotide of the antisense strand is not linked to a 5' phosphate group or a 5' phosphate-derived group, the structure of the 5' end nucleotide is represented by formula X:

formula (X)

wherein Base represents a base, e.g., A, U, G, C, or T; R is hydroxyl or is substituted with various groups known to those skilled in the art; for example, R may be 2'-fluoro (2'-F), 2'-alkoxy, 2'-substituted alkoxy, 2'-alkyl, 2'-substituted alkyl, 2'-amino, 2'-substituted amino, or a 2'-deoxynucleotide.

Illustratively, a structure of the 5' phosphate group is:

;

a structure of the 5' phosphate-derived group includes, but is not limited to:

(EVP),

and the like.

In the case that the ribosyl group at the 5' end nucleotide of the antisense strand has a 5' phosphate group or a 5' phosphate-derived group, a structure as shown below is formed:

, , or ;

wherein Base represents a base, e.g., A, U, G, C, or T. R' is hydroxyl or hydrogen or is substituted with various groups known to those skilled in the art, such as a 2'-fluoro (2'-F)-modified nucleotide, a 2'-alkoxy-modified nucleotide, a 2'-substituted alkoxy-modified nucleotide, a 2'-alkyl-modified nucleotide, a 2'-substituted alkyl-modified nucleotide, or a 2'-deoxyribonucleotide.

In some optional embodiments, the modified siRNA comprises or is selected from the modified siRNAs in Table 1.

In some specific embodiments, an inhibition rate of the modified siRNA of the present disclosure against the ANGPTL3 gene is at least about 20%, and may be at least about 25%, about 30%, about 35%, about 40%, about 45%, about 50%, about 55%, about 60%, about 65%, about 70%, about 75%, about 80%, about 85%, about 90%, about 91%, about 92%, about 93%, about 94%, about 95%, about 96%, about 97%, about 98%, about 99%, or a numerical value or range between any two of these numerical values.

siRNA Conjugate

A second aspect of the present disclosure provides a siRNA conjugate, which is obtained by conjugating the modified siRNA provided in the first aspect of the present disclosure to a conjugated group.

In the present disclosure, the sense strand and the antisense strand of the siRNA conjugate form a double-stranded region of the siRNA conjugate, and a blunt end is formed at the 3' end of the sense strand of the siRNA conjugate. In some embodiments, the 3' end of the sense strand of the siRNA conjugate forms a blunt end, and the 3' end of the antisense strand of the siRNA conjugate has 1-2 overhanging nucleotides extending out of the double-stranded region.

In some preferred embodiments, the siRNA conjugate is obtained by conjugating the modified siRNA to a conjugated group. The sense strand and the antisense strand of the modified siRNA are complementary to form a double-stranded region of the modified siRNA, the 3' end of the sense strand of the modified siRNA forms a blunt end, and the conjugated group is conjugated to the 3' end of the sense strand with the blunt end to form a siRNA conjugate.

Illustratively, the sense strand of the modified siRNA is a sequence as shown in sequence A (or nucleotide sequence I), and the antisense strand is a sequence as shown in sequence B (or nucleotide sequence II) linked to sequence E (or nucleotide sequence IV). In addition, the 3' end of the sense strand of the modified siRNA forms a blunt end, and the 3' end of the sense strand of the modified siRNA is linked to a conjugated group to form a siRNA conjugate.

Illustratively, the sense strand of the modified siRNA is a sequence as shown in sequence A linked to sequence D (or the nucleotide sequence III), and the antisense strand is a sequence as shown in sequence B linked to sequence E. In addition, the 3' end of the sense strand of the modified siRNA has sequence D consisting of 1-2 protruding nucleotides. After the sequence D at the 3' end of the sense strand in the modified siRNA is excluded, a conjugated group is linked to the 3' end of sequence A to form a siRNA conjugate.

Illustratively, the siRNA conjugate represented by N-ER-FY013058M6L96 is a conjugate obtained by N-ER-FY013058M6 and a conjugated group, wherein based on the sequence of N-ER-FY013058M6, it can be known that the 3' end of the sense strand of the conjugate originally has the overhanging nucleotide - sTsT extending out of the double-stranded region, and a sequence containing a blunt end mGsmGsmAmUmUmAUfmCUfUfGfmGmAmAmGmUmCmU-mA formed after the protruding nucleotide -sTsT located at the 3' end of the sense strand is excluded before binding to the conjugated group is used as a nucleotide sequence for linkage with an L96 conjugated group (i.e., linking to L96 via a phosphodiester bond after the sequence is synthesized to the blunt end). Therefore, the sequences for forming the siRNA conjugate are: mGsmGsmAmUmUmAUfmCUfUfGfmGmAmAmGmUmCmUmAL96 (SEQ ID NO: 30) as the sense

strand, and mUsAfsmGmAmCUfmUmCmCmAmAmGmAUfmAAfmUmCmCsTsT (SEQ ID NO: 7) as the antisense strand.

Further, the siRNA molecule linked to the conjugated group in the siRNA conjugate is the modified siRNA. The siRNA molecule modified by the conjugated group has relatively good tissue and organ targeting properties and the ability to promote cellular endocytosis while maintaining relatively high inhibition activity and stability, so that the effect on other tissues or organs can be reduced and the amount of siRNA molecule used can be reduced, thereby achieving the purposes of reducing toxicity and reducing cost. Optionally, any one of the siRNA molecules shown in Table 1 is selected to be linked to the conjugated group to obtain the siRNA conjugate.

The conjugation site between the modified siRNA and the conjugated group may be at the 3' end or 5' end of the sense strand of the modified siRNA, or at the 5' end of the antisense strand, or in an internal sequence of the modified siRNA. In some embodiments, the conjugation site between the modified siRNA and the conjugated group is at the 3' end of the sense strand of the modified siRNA.

In some embodiments, the conjugated group may be linked to the phosphate group, the 2'-position hydroxyl, or the base of the nucleotide. In some embodiments, the conjugated group may be linked to the 3'-position hydroxyl, and at this time, the nucleotides are linked via a 2',5'-phosphodiester bond. When the conjugated group is linked to the end of the modified siRNA chain, the conjugated group is generally linked to the phosphate group of the nucleotide; when the conjugated group is linked to the internal sequence of the modified siRNA, the conjugated group is generally linked to the ribose sugar ring or the base. Various linkage methods can be referred to the publication: Muthiah Manoharan et.al. siRNA conjugates carrying sequentially assembled trivalent N-acetylgalactosamine linked through nucleosides elicit robust gene silencing in vivo in hepatocytes. ACS Chemical biology, 2015, 10(5): 1181-7.

In the present disclosure, the conjugated group may be a ligand conventionally used in the field of siRNA administration. In some embodiments, the conjugated group may be selected from one or more of ligands formed by the following targeting molecules or derivatives thereof: lipophilic molecules such as cholesterol, bile acids, vitamins (e.g., vitamin E), and lipid molecules of different chain lengths; polymers such as polyethylene glycol; polypeptides such as transmembrane peptides; aptamers; antibodies; quantum dots; sugars such as lactose, polylactose, mannose, galactose, and N-acetylgalactosamine (GalNAc); folate; ligands for receptor expressed by hepatic parenchymal cells such as asialoglycoprotein, asialosaccharide residues, lipoproteins (e.g., high-density lipoprotein and low-density lipoprotein), glucagon, neurotransmitters (e.g., epinephrine), growth factors, transferrin, and the like.

In some specific embodiments, the conjugated group has a structure represented by any one of formula I, formula III, formula IV, formula V, formula VI, formula VII, formula VIII, and formula IX described above.

The conjugated group represented by formula I is GalNAc. GalNAc has the liver-targeting property, and can deliver siRNA molecules to liver tissues with high specificity and specifically inhibit the high ANGPTL3 gene expression in the liver.

In some specific embodiments, GalNAc is conjugated to the 3' end of the sense strand via a phosphodiester bond to give a siRNA conjugate with the structure represented by formula II.

In some embodiments, the siRNA conjugate is formed by linking any one of the modified siRNAs shown in Table 1 to a conjugated group;

preferably, in the siRNA conjugate, the sense strand and the antisense strand are selected from the combinations of the sense strand and the antisense strand of the siRNA conjugates shown in Table 2.

In some specific embodiments, an inhibition rate of the siRNA conjugate of the present disclosure against the ANGPTL3 gene is at least about 50%, and may be at least about 55%, about 60%, about 65%, about 70%, about 75%, about 80%, about 85%, about 90%, about 91%, about 92%, about 93%, about 94%, about 95%, about 96%, about 97%, about 98%, about 99%, or a numerical value or range between any two of these numerical values.

Pharmaceutical Composition

A third aspect of the present disclosure provides a pharmaceutical composition, comprising one or more of the modified siRNA according to the first aspect and the siRNA conjugate according to the second aspect.

In some embodiments, the pharmaceutical composition comprises the siRNA (including the modified siRNA and the siRNA conjugate) described above as an active ingredient and a pharmaceutically acceptable carrier. In the present disclosure, the pharmaceutical composition is used for the purpose of promoting the administration to an organism, which facilitates the absorption of the active ingredient so that it can exert its biological activity. The pharmaceutical composition of the present disclosure may be administered in any form, including injection (intraarterial, intravenous, intramuscular, intraperitoneal, or subcutaneous), mucosal, oral (oral solid formulations or oral liquid formulations), rectal, inhalation, implantation, topical (e.g., ocular) administration, and the like. Non-limiting examples of the oral solid formulations include, but are not limited to, powders, capsules, pastilles, granules, tablets, and the like. Non-limiting examples of liquid formulations for oral or mucosal administration include, but are not limited to, suspensions, tinctures, elixirs, solutions, and the like. Non-limiting examples of formulations for topical administration include, but are not limited to, emulsions, gels,

ointments, creams, patches, pastes, foams, lotions, drops, or serum formulations. Non-limiting examples of formulations for parenteral administration include, but are not limited to, solutions for injection, dry powders for injection, suspensions for injection, emulsions for injection, and the like. The pharmaceutical composition of the present disclosure may also be formulated into a controlled-release or delayed-release dosage form (e.g., liposomes or microspheres).

In the present disclosure, the route of administration may be varied or adjusted in any applicable way to meet the needs of the nature of the drug, the convenience of patients and medical personnel, and other relevant factors.

Medical Use

A fourth aspect of the present disclosure provides use of at least one of the following modified siRNAs or siRNA conjugates:

(1) inhibiting ANGPTL3 gene expression, or preparing a medicament for inhibiting ANGPTL3 gene expression;
(2) preventing or treating a disease associated with abnormal ANGPTL3 gene expression, or preparing a medicament for preventing or treating a disease associated with abnormal ANGPTL3 gene expression;
(3) treating a subject suffering from a disease that would benefit from a reduction in ANGPTL3 gene expression, or preparing a medicament for treating a subject suffering from a disease that would benefit from a reduction in ANGPTL3 gene expression.

The present disclosure further provides use of the siRNA molecule (including the modified siRNA and the siRNA conjugate) or the pharmaceutical composition in at least one of (1) to (3) described above.

In the present disclosure, the abnormal ANGPTL3 gene expression causes one or more of the following diseases associated with abnormal ANGPTL3 gene expression: chylomicronemia syndrome, type 2 diabetes, familial partial lipodystrophy, hypercholesterolemia, familial hypercholesterolemia, non-alcoholic fatty liver, atherosclerosis, hypertriglyceridemia, knee injury and osteoarthritis, dyslipidemia (including mixed dyslipidemia), and fatty liver.

The siRNA molecule causes the ANGPTL3 gene expression to be inhibited by at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98% or at least about 99%, achieving treatment of a disease associated with abnormal ANGPTL3 gene expression.

In some embodiments, the present disclosure provides a method for inhibiting intracellular ANGPTL3 gene expression *in vivo* or *in vitro*, comprising contacting a cell expressing ANGPTL3 with, or administering to a subject in need thereof, a therapeutically effective amount of the modified siRNA, the siRNA conjugate, or the pharmaceutical composition.

Further, the method for inhibiting intracellular ANGPTL3 gene expression is to introduce the siRNA molecule (including the modified siRNA and the siRNA conjugate) or the pharmaceutical composition into the cell.

In some embodiments, the cell is an *in vivo* cell or an *in vitro* cell. In some specific embodiments, the cell is in the subject.

In some embodiments, the present disclosure provides a method for preventing or treating a disease associated with abnormal ANGPTL3 gene expression, comprising administering to a subject in need thereof a therapeutically effective amount of the modified siRNA, the siRNA conjugate, or the pharmaceutical composition;

preferably, the disease associated with abnormal ANGPTL3 gene expression is selected from a disease associated with lipid metabolism;
optionally, the disease associated with lipid metabolism is selected from the group consisting of the following diseases:
chylomicronemia syndrome, type 2 diabetes, familial partial lipodystrophy, hypercholesterolemia, familial hypercholesterolemia, non-alcoholic fatty liver, atherosclerosis, hypertriglyceridemia, knee injury and osteoarthritis, dyslipidemia (including mixed dyslipidemia), and fatty liver.

Further, the method for preventing or treating a disease is administering to the subject the siRNA molecule (including the modified siRNA and the siRNA conjugate) or the pharmaceutical composition.

In the present disclosure, "subject" includes humans or non-human animals, preferably vertebrates, and more preferably mammals. The subject may include a transgenic organism. Most preferably, the subject is a human. Further, the subject has at least one of the following characteristics:

(1) abnormal ANGPTL3 gene expression *in vivo*, more specifically, abnormally high ANGPTL3 gene expression;

(2) suffering from a disease associated with abnormal ANGPTL3 gene expression;
(3) suffering from a disease that would benefit from a reduction in ANGPTL3 gene expression. Examples include a person suffering from or predisposed to a disease associated with abnormal ANGPTL3 gene expression.

The amount of the siRNA molecule (including the modified siRNA and the siRNA conjugate) or the pharmaceutical composition of the present disclosure may be determined according to the weight, age, sex, severity of the disease, and the like of the patient. Based on the amount of the siRNA contained therein, the siRNA molecule (including the modified siRNA and the siRNA conjugate) or the pharmaceutical composition of the present disclosure is administered at a dose of about 1-300 mg/kg body weight.

The frequency of administration may be once or more times every day, every week, every two weeks, every three weeks, every month, every 2 months, every 3 months, every 4 months, every 5 months, every 6 months, every 7 months, every 8 months, every 9 months, every 10 months, every 11 months, or every year.

The total number of administrations of the siRNA molecule (including the modified siRNA and the siRNA conjugate) or the pharmaceutical composition of the present disclosure may be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50. For example, the siRNA molecule (including the modified siRNA and the siRNA conjugate) or the pharmaceutical composition of the present disclosure may be administered about 1, 2, 3, or 4 times.

In some embodiments, the siRNA molecule (including the modified siRNA and the siRNA conjugate), the pharmaceutical composition, and optionally another therapeutic agent of the present disclosure may be packaged in a kit, and the siRNA molecule (including the modified siRNA and the siRNA conjugate), a pharmaceutically acceptable carrier, and optionally another therapeutic agent in the kit may be provided in a liquid form or a dry form. In some embodiments, the kit comprises instructions for explaining how to mix the siRNA molecule with the pharmaceutically acceptable carrier or another ingredient.

In some embodiments, the present disclosure provides the modified siRNA, the siRNA conjugate, or the pharmaceutical composition for use in therapy.

Table 1. Sequence information of modified siRNAs

| siRNA No. | siRNA ID | Sense strand Ss (5'->3') | SEQ ID NO: | Antisense strand As (5'->3') | SEQ ID NO: |
|---|---|---|---|---|---|
| siRNA1 | N-ER-FY013058M6 | mGsmGsmAmUmUmAUfmCUfUfGfmGmAmAmGmUmCmUmAsTsT | 1 | mUsAfsmGmAmCUfmUmCmCmAmAmGmAUfmAAfmUmCmCsTsT | 7 |
| siRNA2 | N-ER-FY013058M7 | mGsmGsmAmUmUmAUfmCUfUfGfmGmAmAmGmUmCmUmAsTsT | 1 | mUsAfsmGmAmCUfmUCfCfmAmAmGmAUfmAAfmUmCmCsTsT | 8 |
| siRNA3 | N-ER-FY013058M8 | mGsmGsmAmUmUmAUfmCUfUfGfmGmAmAmGmUmCmUmAsTsT | 1 | mUsAfsmGmAmC[**GNA**]UmUmCmCmAmAmGmAUfmAAfmUmCmCsTsT | 9 |
| siRNA4 | N-ER-FY013058M9 | mGsmGsmAmUmUmAUfmCUfUfGfmGmAmAmGmUmCmUmAsTsT | 1 | mUsAfsmGmAmCUf[**GNA**]UmCmCmAmAmGmAUfmAAfmUmCmCsTsT | 10 |
| siRNA5 | N-ER-FY013060M6 | mGsmUsmCmUmCmAAfmAAfUfGfmGmAmAmGmGmUmUmAsTsT | 2 | mUsAfsmAmCmCUfmUmCmCmAmUmUmUUfmGAfmGmAmCsTsT | 11 |
| siRNA6 | N-ER-FY013060M7 | mGsmUsmCmUmCmAAfmAAfUfGfmGmAmAmGmGmUmUmAsTsT | 2 | mUsAfsmAmCmCUfmUCfCfmAmUmUmUUfmGAfmGmAmCsTsT | 12 |

(continued)

| siRNA No. | siRNA ID | Sense strand Ss (5'->3') | SEQ ID NO: | Antisense strand As (5'->3') | SEQ ID NO: |
|---|---|---|---|---|---|
| siRNA7 | N-ER-FY013060M8 | mGsmUsmCmUmCmAAfmAAfUfGfmGmAmAmGmGmUmUmAsTsT | 2 | mUsAfsmAmCmC[**GNA**]UmUmCmCmAmUmUmUUfmGAfmGmAmCsTsT | 13 |
| siRNA8 | N-ER-FY013060M9 | mGsmUsmCmUmCmAAfmAAfUfGfmGmAmAmGmGmUmUmAsTsT | 2 | mUsAfsmAmCmCUf[**GNA**]UmCmCmAmUmUmUUfmGAfmGmAmCsTsT | 14 |
| siRNA9 | N-ER-FY013062M6 | mGsmGsmAmAmGmGUfmUAfUfAfmCmUmCmUmAmUmAmAsTsT | 3 | mUsUfsmAmUmAGfmAmGmUmAmUmAmACfmCUfmUmCmCsTsT | 15 |
| siRNA10 | N-ER-FY013062M6D2 | mGsmGsmAmAmGmGUfmUAfUfAfmCmUmCmUmAmUmAmA | 4 | mUsUfsmAmUmAGfmAmGmUmAmUmAmACfmCUfmUmCmCsTsT | 15 |
| siRNA11 | N-ER-FY013062M7 | mGsmGsmAmAmGmGUfmUAfUfAfmCmUmCmUmAmUmAmAsTsT | 3 | mUsUfsmAmUmAGfmAGfUfmAmUmAmACfmCUfmUmCmCsTsT | 16 |
| siRNA12 | N-ER-FY013062M7D2 | mGsmGsmAmAmGmGUfmUAfUfAfmCmUmCmUmAmUmAmA | 4 | mUsUfsmAmUmAGfmAGfUfmAmUmAmACfmCUfmUmCmCsTsT | 16 |
| siRNA13 | N-ER-FY013062M8 | mGsmGsmAmAmGmGUfmUAfUfAfmCmUmCmUmAmUmAmAsTsT | 3 | mUsUfsmAmUmA[**GNA**]GmAmGmUmAmUmAmACfmCUfmUmCmCsTsT | 17 |
| siRNA14 | N-ER-FY013062M9 | mGsmGsmAmAmGmGUfmUAfUfAfmCmUmCmUmAmUmAmAsTsT | 3 | mUsUfsmAmUmAGf[**GNA**]AmGmUmAmUmAmACfmCUfmUmCmCsTsT | 18 |
| siRNA15 | N-ER-FY013139M6 | mGsmGsmAmAmGmGUfmUAfUfAfmCmUmCmUmAmUmAmUsTsT | 5 | mAsUfsmAmUmAGfmAmGmUmAmUmAmACfmCUfmUmCmCsTsT | 19 |
| siRNA16 | N-ER-FY013139M7 | mGsmGsmAmAmGmGUfmUAfUfAfmCmUmCmUmAmUmAmUsTsT | 5 | mAsUfsmAmUmAGfmAGfUfmAmUmAmACfmCUfmUmCmCsTsT | 20 |
| siRNA17 | N-ER-FY013139M8 | mGsmGsmAmAmGmGUfmUAfUfAfmCmUmCmUmAmUmAmUsTsT | 5 | mAsUfsmAmUmA[**GNA**]GmAmGmUmAmUmAmACfmCUfmUmCmCsTsT | 21 |
| siRNA18 | N-ER-FY013139M9 | mGsmGsmAmAmGmGUfmUAfUfAfmCmUmCmUmAmUmAmUsTsT | 5 | mAsUfsmAmUmAGf[**GNA**]AmGmUmAmUmAmACfmCUfmUmCmCsTsT | 22 |
| siRNA19 | N-ER-FY013140M6 | mGsmUsmCmUmCmAAfmAAfUfGfmGmAmAmGmGmUmUmUsTsT | 6 | mAsAfsmAmCmCUfmUmCmCmAmUmUmUUfmGAfmGmAmCsTsT | 23 |

(continued)

| siRNA No. | siRNA ID | Sense strand Ss (5'->3') | SEQ ID NO: | Antisense strand As (5'->3') | SEQ ID NO: |
|---|---|---|---|---|---|
| siRNA20 | N-ER-FY013140M7 | mGsmUsmCmUmCmAAfmAAfUfGfmGmAmAmGmGmUmUmUsTsT | 6 | mAsAfsmAmCmCUfmUCfCfmAmUmUmUUfmGAfmGmAmCsTsT | 24 |
| siRNA21 | N-ER-FY013140M8 | mGsmUsmCmUmCmAAfmAAfUfGfmGmAmAmGmGmUmUmUsTsT | 6 | mAsAfsmAmCmC[**GNA**]UmUmCmCmAmUmUmUUfmGAfmGmAmCsTsT | 25 |
| siRNA22 | N-ER-FY013140M9 | mGsmUsmCmUmCmAAfmAAfUfGfmGmAmAmGmGmUmUmUsTsT | 6 | mAsAfsmAmCmCUf[**GNA**]UmCmCmAmUmUmUUfmGAfmGmAmCsTsT | 26 |

The uppercase letters "G", "C", "A", "T", and "U" in the table described above generally represent nucleotides containing guanine, cytosine, adenine, thymine, and uracil as bases, respectively; mA, mU, mC, and mG represent 2'-methoxy-modified nucleotides; Af, Gf, Cf, and Uf represent 2'-fluoro-modified nucleotides; the lowercase letter s represents that the two nucleotides adjacent to the letter s are linked via a phosphorothioate diester bond; [**GNA**] represents that one ribonucleotide adjacent to its right side is a ribonucleotide with a GNA modification.

Table 2. Sequence information of siRNA conjugates

| siRNA No. | siRNA ID | Modified sense strand Ss linked to conjugated group (5'->3') | SEQ ID NO: | Modified antisense strand As (5'->3') | SEQ ID NO: |
|---|---|---|---|---|---|
| siRNA23 | N-ER-FY013058M6L96 | mGsmGsmAmUmUmAUfmCUfUfGfmGmAmAmGmUmCmUmAL96 | 30 | mUsAfsmGmAmCUfmUmCmCmAmAmGmAUfmAAfmUmCmCsTsT | 7 |
| siRNA24 | N-ER-FY013058M7L96 | mGsmGsmAmUmUmAUfmCUfUfGfmGmAmAmGmUmCmUmAL96 | 30 | mUsAfsmGmAmCUfmUCfCfmAmAmGmAUfmAAfmUmCmCsTsT | 8 |
| siRNA25 | N-ER-FY013058M8L96 | mGsmGsmAmUmUmAUfmCUfUfGfmGmAmAmGmUmCmUmAL96 | 30 | mUsAfsmGmAmC[**GNA**]UmUmCmCmAmAmGmAUfmAAfmUmCmCsTsT | 9 |
| siRNA26 | N-ER-FY013058M9L96 | mGsmGsmAmUmUmAUfmCUfUfGfmGmAmAmGmUmCmUmAL96 | 30 | mUsAfsmGmAmCUf[**GNA**]UmCmCmAmAmGmAUfmAAfmUmCmCsTsT | 10 |
| siRNA27 | N-ER-FY013060M6L96 | mGsmUsmCmUmCmAAfmAAfUfGfmGmAmAmGmGmUmUmAL96 | 31 | mUsAfsmAmCmCUfmUmCmCmAmUmUmUUfmGAfmGmAmCsTsT | 11 |
| siRNA28 | N-ER-FY013060M7L96 | mGsmUsmCmUmCmAAfmAAfUfGfmGmAmAmGmGmUmUmAL96 | 31 | mUsAfsmAmCmCUfmUCfCfmAmUmUmUUfmGAfmGmAmCsTsT | 12 |
| siRNA29 | N-ER-FY013060M8L96 | mGsmUsmCmUmCmAAfmAAfUfGfmGmAmAmGmGmUmUmAL96 | 31 | mUsAfsmAmCmC[**GNA**]UmUmCmCmAmUmUmUUfmGAfmGmAmCsTsT | 13 |

(continued)

| siRNA No. | siRNA ID | Modified sense strand Ss linked to conjugated group (5'->3') | SEQ ID NO: | Modified antisense strand As (5'->3') | SEQ ID NO: |
|---|---|---|---|---|---|
| siRNA30 | N-ER-FY013060M9L96 | mGsmUsmCmUmCmAAfmAAfUfGfmGmAmAmGmGmUmUmAL96 | 31 | mUsAfsmAmCmCUf[**GNA**]UmCmCmAmUmUmUUfmGAfmGmAmCsTsT | 14 |
| siRNA31 | N-ER-FY013062M6L96 | mGsmGsmAmAmGmGUfmUAfUfAfmCmUmCmUmAmUmAmAL96 | 32 | mUsUfsmAmUmAGfmAmGmUmAmUmAmACfmCUfmUmCmCsTsT | 15 |
| siRNA32 | N-ER-FY013062M7L96 | mGsmGsmAmAmGmGUfmUAfUfAfmCmUmCmUmAmUmAmAL96 | 32 | mUsUfsmAmUmAGfmAGfUfmAmUmAmACfmCUfmUmCmCsTsT | 16 |
| siRNA33 | N-ER-FY013062M8L96 | mGsmGsmAmAmGmGUfmUAfUfAfmCmUmCmUmAmUmAmAL96 | 32 | mUsUfsmAmUmA[**GNA**]GmAmGmUmAmUmAmACfmCUfmUmCmCsTsT | 17 |
| siRNA34 | N-ER-FY013062M9L96 | mGsmGsmAmAmGmGUfmUAfUfAfmCmUmCmUmAmUmAmAL96 | 32 | mUsUfsmAmUmAGf[**GNA**]AmGmUmAmUmAmACfmCUfmUmCmCsTsT | 18 |
| siRNA35 | N-ER-FY013139M6L96 | mGsmGsmAmAmGmGUfmUAfUfAfmCmUmCmUmAmUmAmUL96 | 33 | mAsUfsmAmUmAGfmAmGmUmAmUmAmACfmCUfmUmCmCsTsT | 19 |
| siRNA36 | N-ER-FY013139M7L96 | mGsmGsmAmAmGmGUfmUAfUfAfmCmUmCmUmAmUmAmUL96 | 33 | mAsUfsmAmUmAGfmAGfUfmAmUmAmACfmCUfmUmCmCsTsT | 20 |
| siRNA37 | N-ER-FY013139M8L96 | mGsmGsmAmAmGmGUfmUAfUfAfmCmUmCmUmAmUmAmUL96 | 33 | mAsUfsmAmUmA[**GNA**]GmAmGmUmAmUmAmACfmCUfmUmCmCsTsT | 21 |
| siRNA38 | N-ER-FY013139M9L96 | mGsmGsmAmAmGmGUfmUAfUfAfmCmUmCmUmAmUmAmUL96 | 33 | mAsUfsmAmUmAGf[**GNA**]AmGmUmAmUmAmACfmCUfmUmCmCsTsT | 22 |
| siRNA39 | N-ER-FY013140M6L96 | mGsmUsmCmUmCmAAfmAAfUfGfmGmAmAmGmGmUmUmUL96 | 34 | mAsAfsmAmCmCUfmUmCmCmAmUmUmUUfmGAfmGmAmCsTsT | 23 |
| siRNA40 | N-ER-FY013140M7L96 | mGsmUsmCmUmCmAAfmAAfUfGfmGmAmAmGmGmUmUmUL96 | 34 | mAsAfsmAmCmCUfmUCfCfmAmUmUmUUfmGAfmGmAmCsTsT | 24 |
| siRNA41 | N-ER-FY013140M8L96 | mGsmUsmCmUmCmAAfmAAfUfGfmGmAmAmGmGmUmUmUL96 | 34 | mAsAfsmAmCmC[**GNA**]UmUmCmCmAmUmUmUUfmGAfmGmAmCsTsT | 25 |
| siRNA42 | N-ER-FY013140M9L96 | mGsmUsmCmUmCmAAfmAAfUfGfmGmAmAmGmGmUmUmUL96 | 34 | mAsAfsmAmCmCUf[**GNA**]UmCmCmAmUmUmUUfmGAfmGmAmCsTsT | 26 |

The uppercase letters "G", "C", "A", "T", and "U" in the table described above generally represent nucleotides containing guanine, cytosine, adenine, thymine, and uracil as bases, respectively; mA, mU, mC, and mG represent 2'-methoxy-modified nucleotides; Af, Gf, Cf, and Uf represent 2'-fluoro-modified nucleotides; the lowercase letter s represents that the two nucleotides adjacent to the letter s are linked via a phosphorothioate diester bond; [**GNA**] represents that one ribonucleotide adjacent to its right side is a ribonucleotide with a GNA modification; L96 is the conjugate group GalNAc represented by formula I.

In Tables 1 and 2, the 5' end nucleotides of the modified sense strand and the modified sense strand linked to the conjugated group are not linked to a 5' phosphate group or a 5' phosphate-derived group, and their structures are represented by formula X:

HO Base O R O formula (X)

wherein Base represents a base, e.g., A, U, G, C, or T; R is hydroxyl or is substituted with various groups known to those skilled in the art; for example, R may be 2'-fluoro (2'-F), 2'-alkoxy, 2'-substituted alkoxy, 2'-alkyl, 2'-substituted alkyl, 2'-amino, 2'-substituted amino, or a 2'-deoxynucleotide.

In Tables 1 and 2, the 5' end nucleotide of the modified antisense strand is not linked to a 5' phosphate group or a 5' phosphate-derived group, and its structure is also represented by formula X.

In Tables 1 and 2, the 3' positions of the 3' end nucleotides of the modified sense strand and the modified antisense strand are hydroxyl groups.

Example

Other objects, features, and advantages of the present disclosure will become apparent from the following detailed description. However, it should be understood that the detailed description and the specific examples, while indicating specific embodiments of the present disclosure, are provided for explanatory purposes only. After reading this detailed description, various changes and modifications made within the spirit and scope of the present disclosure will become apparent to those skilled in the art.

The experimental techniques and methods used in the examples, unless otherwise specified, are all conventional techniques and methods. For example, in the following examples, if no specific conditions are indicated, the experimental methods are generally performed under conventional conditions, such as those described in Sambrook et al., Molecular Cloning: A Laboratory Manual (New York: Cold Spring Harbor Laboratory Press, 1989) or those as recommended by the manufacturer. All materials, reagents, and the like used in the examples are commercially available unless otherwise specified.

The modified siRNAs and siRNA conjugates involved in the following examples were synthesized by Shanghai Talen-bio Scientific Co., Ltd. The cells and reagents used in the following examples are shown in Table 3:

Table 3

| Name | Supplier | Cat. No. |
|---|---|---|
| Huh7 | JCRB cell bank | JCRB0403 |
| RNeasy®96Kit | QIAGEN | QIAGEN-74182 |
| Lipo®RNAiMAX transfection reagent | Invitrogen | 13778-150 |
| Opti-medium | Gibco | 31985-070 |
| Fetal Bovine Serum | ExCellBio | FSP500 |
| Phosphate Buffered Saline (PBS) | Gibco | 10010-023 |
| 0.05%Trypsin-EDTA | Gibco | 25300-062 |
| Fastking RT Kit (with gDNase) | TianGen | KR116-02 |
| FastStart universal probe master(ROX) | Roche | 04914058001 |
| TaqMan probe/primer (GAPDH) | Thermo | 4351268 Assay ID: Hs02786624_g1 |

(continued)

| Name | Supplier | Cat. No. |
|---|---|---|
| TaqMan probe/primer (ANGPTL3) | Thermo | 4351268 Assay ID: Hs00205581_m1 |

Example 1: Synthesis of siRNAs

1.1 Description of synthesis method:

Nucleoside monomers were linked one by one in the 3'-5' direction in the order according to the nucleotide arrangement by a solid-phase phosphoramidite method. The linkage of each nucleoside monomer involves four reactions: deprotection, coupling, oxidation or sulfurization, and capping. When a phosphoester linkage is present between two nucleotides, the steps for linking the subsequent nucleoside monomer include deprotection, coupling, oxidation, and capping. When a phosphorothioate diester bond linkage is present between two nucleotides, the steps for linking the subsequent nucleoside monomer include deprotection, coupling, sulfurization, and capping. In the present disclosure, nucleotide monomers are selected according to the target sequence to be synthesized, and the selected nucleotide monomers are nucleotide monomers commonly used by those skilled in the art. For example, the nucleotide monomer for synthesizing A may be, but is not limited to, adenosine-3-phosphate. It should be understood that these monomers, when present in the oligonucleotide, are linked to each other via a 5'-3' phosphodiester bond or a 5'-3' phosphorothioate diester bond, and in cases where, for example, the last nucleotide in the 5' to 3' direction has a hydroxyl group at its 3' position, this is achieved according to conventional techniques in the art.

1.2 Given synthesis conditions:

Nucleoside monomers were provided in an acetonitrile solution with a concentration of 0.1 M. The conditions for the deprotection reaction in each step were the same: the temperature was 25 °C; the reaction time was 70 s; the deprotection reagent was a solution (3% V/V) of dichloroacetic acid in dichloromethane; and the molar ratio of dichloroacetic acid to the 4,4'-dimethoxytrityl protecting group on the solid-phase support was 5:1.

The conditions for the coupling reaction in each step were the same, which included: the temperature was 25 °C; the reaction time was 600 s; the coupling reagent was a 0.5 M solution of 5-ethylthio-1H-tetrazole in acetonitrile; the molar ratio of the nucleic acid sequence linked to the solid-phase support to the nucleoside monomer was 1:10; and the molar ratio of the nucleic acid sequence linked to the solid-phase support to the coupling reagent was 1:65.

The conditions for the oxidation reaction in each step were the same, which included: the temperature was 25 °C; the reaction time was 15 s; the oxidation reagent was iodine water with a concentration of 0.05 M. The molar ratio of the iodine to the nucleic acid sequence linked to the solid-phase support in the coupling step was 30:1. The reaction was carried out in a mixed solvent of tetrahydrofuran:water:pyridine = 3:1:1.

The conditions for the sulfurization reaction in each step were the same, which included: the temperature was 25 °C; the reaction time was 300 s; the sulfurizing reagent was xanthane hydride. The molar ratio of the sulfurizing reagent to the nucleic acid sequence linked to the solid-phase support in the coupling step was 120:1. The reaction was carried out in a mixed solvent of acetonitrile:pyridine = 1:1.

The conditions for the capping reaction in each step were the same, which included: the temperature was 25 °C, and the reaction time was 15 s. The capping reagent solution was a mixed solution of CapA and CapB at a molar ratio of 1:1, and the molar ratio of the capping reagent to the nucleic acid sequence linked to the solid-phase support was acetic anhydride:N-methylimidazole:nucleic acid sequence linked to the solid-phase support = 1:1:1.

After the last nucleoside monomer was linked, the nucleic acid sequence linked to the solid-phase support was sequentially subjected to ammonolysis, purification, desalting, and lyophilization to obtain a sense strand and an antisense strand. Finally, the two strands were heated and annealed to obtain a product, which was then lyophilized to obtain a lyophilized powder.

The synthesized modified siRNAs are shown in Table 1.

Example 2: Synthesis of siRNA Conjugates (GalNAc-siRNAs)

2.1 siRNA conjugates with structure represented by formula II below:

formula II

2.2 Synthesis process of siRNA conjugates

Step 1: reaction of DMTr-L96 with succinic anhydride to obtain compound L96-A:

DMTr-L96

L96-A

Preparation process: DMTr-L96, succinic anhydride, 4-dimethylaminopyridine, and diisopropylethylamine were added to dichloromethane, and the mixture was reacted under stirring at 25 °C for 24 h. The reaction liquid was then washed with 0.5 M triethylammonium phosphate. The aqueous phase was washed three times with dichloromethane, and the organic phases were combined and concentrated under reduced pressure to obtain a crude product. The crude product was purified by column chromatography to obtain pure L96-A.

Step 2: reaction of L96-A with $NH_2$-SPS to obtain L96-B:

L96-A

L96-B

Preparation process: L96-A, O-(benzotriazol-1-yl)-N,N,N',N'-tetramethyluronium hexafluorophosphate (HBTU), and diisopropylethylamine (DIPEA) were mixed and dissolved in acetonitrile. The mixture was stirred at room temperature for 5 min to obtain a homogeneous solution. An aminomethyl resin ($NH_2$-SPS, 100-200 mesh) was added to the reaction liquid. The mixture was reacted on a shaker at 25 °C for 18 h and then filtered, and the filter cake was washed sequentially with dichloromethane and acetonitrile to obtain a filter cake. The resulting filter cake was subjected to a capping reaction using a CapA/CapB mixed solution to obtain L96-B, which served as the solid-phase support containing the conjugated molecule. Subsequently, nucleoside monomers were linked to the conjugated molecule under a coupling reaction. The siRNA sense strand linked to the conjugate molecule was then synthesized according to the previously described siRNA molecule synthesis method, and the siRNA antisense strand was synthesized using the previously described siRNA molecule synthesis method. Finally, annealing was performed to generate the siRNA conjugate of the present disclosure.

The synthesized siRNA conjugates are shown in Table 2.

Example 3. Inhibition of ANGPTL3 Gene Expression by modified siRNAs

3.1 Experimental materials:

Huh7 cells, JCRB cell bank, Cat. No. JCRB0403;
RNA extraction kit, RNeasy® 96Kit, Cat. No. QIAGEN-74182;
Lipo®RNAiMAX transfection reagent, purchased from Invitrogen, Cat. No. 13778-150;
Opti-medium: reduced serum culture medium, purchased from Gibco, Cat. No. 31985-070;
Fastking RT Kit (with gDNase), purchased from TianGen, Cat. No. KR116-02.

3.2 Experimental methods:

3.2.1 Huh7 cells were taken, washed with PBS, digested with trypsin, and adjusted to a cell density of $5.5 \times 10^5$ cells/mL. Then the cells were seeded into a 96-well plate at a density of 20,000 cells/well, with 100 μL of the culture solution in each well. Huh7 cells were incubated overnight in an incubator at 37 °C with 5% $CO_2$.

3.2.2 The dry powder of the modified siRNA to be tested (collectively referred to as siRNA in the description of the experimental process in this example for convenience of description) was subjected to low-temperature high-speed centrifugation, and then dissolved in ultrapure distilled water to prepare a 100 μM siRNA stock solution.

3.2.3 Preparation of 2 nM siRNA dilution Z and 0.2 nM siRNA dilution W

(1) Preparation of 0.1 μM siRNA reserve solution X and 0.01 μM siRNA reserve solution Y:

a) 2 μL of the 100 μM siRNA stock solution prepared in step 3.2.2 above was taken, and 18 μL of ultrapure distilled water was added to obtain a siRNA dilution with a final concentration of 10 μM;
b) 2 μL of the 10 μM siRNA dilution prepared in step a) was taken, and 18 μL of ultrapure distilled water was added to obtain a siRNA dilution with a final concentration of 1 μM;
c) 2 μL of the 1 μM siRNA dilution prepared in step b) was taken, and 18 μL of ultrapure distilled water was added to obtain a siRNA reserve solution X with a final concentration of 0.1 μM;
d) 2 μL of the 0.1 μM siRNA reserve solution X prepared in step c) was taken, and 18 μL of ultrapure distilled water was added to obtain a siRNA reserve solution Y with a final concentration of 0.01 μM;

(2) 2 μL of each of the prepared siRNA reserve solution X and siRNA reserve solution Y was taken, and 98 μL of Opti-medium was added to obtain a 2 nM siRNA dilution Z and a 0.2 nM siRNA dilution W, respectively.

3.2.4 Transfection of Huh7 cells

(1) 3 μL of the Lipo®RNAiMAX transfection reagent was taken, and 97 μL of Opti-medium was added to obtain a Lipo®RNAiMAX transfection reagent dilution. The Lipo®RNAiMAX transfection reagent dilution was mixed with the 2 nM siRNA dilution Z prepared in step 3.2.3 in a volume ratio of 1:1 to prepare a transfection mixture, which was left to stand for 5 min. 10 μL of the transfection mixture was added to a 96-well plate to transfect the Huh7 cells cultured in step 3.2.1 (the final volume was 100 μL, and the concentration of siRNA in the system was 0.1 nM);
(2) 3 μL of Lipo®RNAiMAX transfection reagent was taken, and 97 μL of Opti-medium was added to obtain a Lipo®RNAiMAX transfection reagent dilution. The Lipo®RNAiMAX transfection reagent dilution was mixed with the 0.2 nM siRNA dilution W prepared in step 3.2.3 in a volume ratio of 1:1 to prepare a transfection mixture, which was left to stand for 5 min. 10 μL of the transfection mixture was added to a 96-well plate to transfect the Huh7 cells cultured in step 3.2.1 (the final volume was 100 μL, and the concentration of siRNA in the system was 0.01 nM).

The cells were cultured for 24 h after the transfection described above, with 2 replicates set for each concentration (0.1 nM and 0.01 nM).

3.2.5 The total RNA of the Huh7 cells obtained in step 3.2.4 was extracted according to the product instructions of the RNeasy® 96 Kit.

3.2.6. The extracted total RNA was reverse-transcribed into cDNA using the Fastking RT Kit (with gDNase), and the reverse transcription was performed according to the following steps:
a) gDNA was removed with the gDNA enzyme according to the table below:

Table 4

| | Volume/μL |
|---|---|
| 5× gDNA Buffer | 2 |
| Sample ( RNA) | 8 |

42 °C, 3 min; 4 °C, standing.

b) The reverse transcription procedure was performed as described below:

Table 5

| | Volume/μL |
|---|---|
| FastKing RT Enzyme Mix | 1 |
| FQ-RT Primer Mix | 2 |
| 10×King RT Buffer | 2 |
| RNase-Free ddH$_2$O | 5 |

42 °C, 15 min; 95 °C, 3 min.

c) The reverse transcription product was stored at -20 °C for real-time PCR analysis.

3.2.7 Real-time PCR analysis with FastStart universal probe master (ROX)

a) The qPCR reaction mixture was prepared as shown in the table below, all reagents were placed on ice throughout the operation;

Table 6

| | Volume/μL |
|---|---|
| 2 ×FastStart universal probe master | 5 |
| 20× target special gene TaqMan probe/primer (ANGPTL3) | 0.5 |
| 20× target special gene TaqMan probe/primer (GAPDH) | 0.5 |
| cDNA obtained in step 3.2.6 and H$_2$O | 4 |
| Total volume | 10 |

b) The qPCR procedure was performed as described below:

95 °C, 10 min;
95 °C, 15 s; 60 °C, 1 min (40 cycles).

3.2.8 Results analysis

a) The Ct value was automatically calculated using the Quant Studio 7 software with default settings;
b) the relative expression level of the gene was calculated using the following formula:

$$\Delta Ct = Ct\ (ANGPTL3\ gene) - Ct\ (GAPDH)$$

$$\Delta\Delta Ct = \Delta Ct\ (test\ sample\ group) - \Delta Ct\ (Mock\ group)$$

$$mRNA\ expression\ relative\ to\ Mock\ group = 2^{-\Delta\Delta Ct},$$

where the Mock group represents a group to which no siRNA was added as compared with the test sample group.

Inhibition rate (%) = (relative expression level of mRNA in Mock group - relative expression level of mRNA in test sample group)/relative expression level of mRNA in Mock group × 100%.

3.3 Silencing experiment results

Testing was performed at concentrations of 0.1 nM and 0.01 nM, and the results are shown in Table 7.

Table 7

| siRNA ID | 0.01 nM -24 h (%) | 0.1 nM -24 h (%) |
|---|---|---|
| N-ER-FY013058M6 | 38.61 | 69.23 |
| N-ER-FY013058M7 | 39.66 | 73.12 |
| N-ER-FY013058M8 | 40.13 | 65.18 |
| N-ER-FY013058M9 | 39.67 | 64.21 |
| N-ER-FY013060M6 | 40.88 | 70.17 |
| N-ER-FY013060M7 | 43.27 | 71.25 |
| N-ER-FY013060M8 | 36.57 | 67.21 |
| N-ER-FY013060M9 | 30.55 | 54.31 |
| N-ER-FY013062M6 | 40.28 | 86.26 |
| N-ER-FY013062M6D2 | 35.62 | 83.77 |
| N-ER-FY013062M7 | 35.55 | 85.16 |
| N-ER-FY013062M7D2 | 35.76 | 83.55 |
| N-ER-FY013062M8 | 26.60 | 65.17 |
| N-ER-FY013062M9 | 24.80 | 58.78 |
| N-ER-FY013139M6 | 29.64 | 69.25 |
| N-ER-FY013139M7 | 32.96 | 70.88 |
| N-ER-FY013139M8 | 21.60 | 67.43 |
| N-ER-FY013139M9 | 23.51 | 63.71 |
| N-ER-FY013140M6 | 38.63 | 66.78 |
| N-ER-FY013140M7 | 36.27 | 77.45 |
| N-ER-FY013140M8 | 34.47 | 70.11 |
| N-ER-FY013140M9 | 33.38 | 72.74 |

As can be seen from Table 7, the modified siRNAs provided in the present disclosure show excellent inhibition effects on the ANGPTL3 gene. At a concentration of 0.01 nM, the inhibition rate at 24 h can reach about 35% or more; at a concentration of 0.1 nM, the inhibition rate at 24 h can reach about 80% or more.

Example 4: Determination of Inhibition Rates of siRNA Conjugates against ANGPTL3 Gene Expression

4.1 Experimental materials:

Primary human hepatocyte (PHH) cells, provided by WuXi AppTec (Shanghai) Co., Ltd;
PHH culture medium: invitroGRO CP Meduim serum free BIOVIT, Cat. No.: S03316;
Lipo®RNAiMAX transfection reagent, purchased from Invitrogen, Cat. No.: 13778-150;
RNA extraction kit RNeasy® 96 Kit, purchased from QIAGEN, Cat. No.: QIAGEN-74182;
Reverse transcription kit FastKing RT Kit (with gDNase), purchased from TianGen, Cat. No.: KR116-02;
FastStart Universal Probe master (ROX), purchased from Roche, Cat. No.: 04914058001;
Primers for ANGPTL3 and GAPDH were provided by WuXi AppTec (Shanghai) Co., Ltd.

4.2 Experimental methods:

siRNA conjugates (final concentrations of siRNA conjugates were 5 nM and 0.5 nM, with replicates) were introduced

into PHH cells via transfection, and the procedures were as described below: Frozen PHH cells were taken, thawed, counted, and adjusted to $6 \times 10^5$ cells/mL. Simultaneously, the siRNA conjugates were introduced into the cells via transfection using the Lipo®RNAiMAX transfection reagent, and the cells were seeded into a 96-well plate at a density of 54,000 cells per well, with 100 μL of the culture solution in each well. The cells were cultured in an incubator at 37 °C with 5% $CO_2$. After 48 h, the culture medium was removed, and the cells were collected for total RNA extraction. The total RNA was extracted using the RNeasy® 96 Kit according to the kit instructions.

siRNA conjugates (final concentrations of siRNA conjugates were 100 nM and 10 nM, with replicates) were introduced into PHH cells via free uptake, and the procedures were as described below: Frozen PHH cells were taken, thawed, counted, and adjusted to $6 \times 10^5$ cells/mL. Simultaneously, the siRNA conjugates were added, and the cells were seeded into a 96-well plate at a density of 54,000 cells per well, with 100 μL of the culture solution in each well. The cells were cultured in an incubator at 37 °C with 5% $CO_2$. After 48 h, the culture medium was removed, and the cells were collected for total RNA extraction. The total RNA was extracted using the RNeasy® 96 Kit according to the kit instructions.

The extracted total RNA was reverse transcribed into cDNA by a reverse transcription reaction using a method similar to that in Example 3. ANGPTL3 cDNA was analyzed by qPCR. GAPDH cDNA was analyzed in parallel as an internal control. The PCR reaction program was: 95 °C for 10 min, followed by a cycling mode of 95 °C for 15 s and then 60 °C for 60 s, for a total of 40 cycles.

Results analysis:

a) The Ct value was automatically calculated using the Quant Studio 7 software with default settings;

b) the relative expression level of the gene was calculated using the following formula:

$$\Delta Ct = Ct\ (ANGPTL3\ gene) - Ct\ (GAPDH)$$

$\Delta\Delta Ct = \Delta Ct$ (test sample group) - $\Delta Ct$ (Mock group), where the Mock group represents a group to which no siRNA conjugate was added as compared with the test sample group;

$$\text{mRNA expression relative to Mock group} = 2^{-\Delta\Delta Ct}$$

Inhibition rate (%) = (relative expression level of mRNA in Mock group - relative expression level of mRNA in test sample group)/relative expression level of mRNA in Mock group × 100%.

Table 8

| siRNA ID | Inhibition rate (%)-free uptake | | Inhibition rate (%)-transfection | |
|---|---|---|---|---|
| | 100 nM | 10 nM | 5 nM | 0.5 nM |
| N-ER-FY013058M6L96 | 76.33 | 67.21 | 76.31 | 67.34 |
| N-ER-FY013058M7L96 | 84.53 | 75.18 | 84.56 | 71.27 |
| N-ER-FY013058M8L96 | 73.22 | 61.30 | 78.26 | 60.05 |
| N-ER-FY013058M9L96 | 63.28 | 54.35 | 78.17 | 52.18 |
| N-ER-FY013060M6L96 | 74.17 | 60.30 | 72.21 | 61.36 |
| N-ER-FY013060M7L96 | 73.17 | 65.27 | 78.24 | 62.19 |
| N-ER-FY013060M8L96 | 73.21 | 62.78 | 76.22 | 60.31 |
| N-ER-FY013060M9L96 | 71.44 | 63.78 | 76.21 | 63.05 |
| N-ER-FY013062M6L96 | 86.90 | 79.56 | 87.62 | 77.48 |
| N-ER-FY013062M7L96 | 74.38 | 62.16 | 77.05 | 66.31 |
| N-ER-FY013062M8L96 | 71.26 | 62.37 | 74.09 | 58.21 |
| N-ER-FY013062M9L96 | 71.05 | 60.28 | 76.13 | 65.07 |

As can be seen from Table 8, the siRNA conjugates provided in the present disclosure show excellent inhibition

effects on the ANGPTL3 gene. In free-uptake conditions, the inhibition rate was up to 86.90% at a concentration of 100 nM and up to 79.56% at a concentration of 10 nM; in transfection conditions, the inhibition rate was up to 87.62% at a concentration of 5 nM and up to 77.48% at a concentration of 0.5 nM.

Example 5: Determination of Inhibition Rates of siRNA Conjugates against ANGPTL3 Gene Expression and Stability Study

5.1 The inhibition rates of the ANGPTL3 gene expression by N-ER-FY013062M2L96 (as shown in Table 9, the synthetic method was according to Example 2) and N-ER-FY013062M6L96 in both free uptake and transfection modes were tested according to Example 4. The results are shown in Table 10.

Table 9

| siRNA ID | Modified sense strand linked to conjugated group (5'->3') | SEQ ID NO: | Modified antisense strand (5'->3') | SEQ ID NO: |
|---|---|---|---|---|
| N-ER-FY013062M2L96 | mGsmGsmAmAmGmGUfmUAfUfAfmCmUmCmUmAmUmAmAL96 | 32 | P1mUsUfsmAmUmAGfmAmGmUmAmUmAmACfmCUfmUmCmCsTsT | 29 |

In Table 9, P1 represents that one nucleotide adjacent to the right side of P1 is a 5'-phosphate nucleotide.

Table 10

| siRNA ID | Inhibition rate (%)-free uptake | | Inhibition rate (%)-transfection | |
|---|---|---|---|---|
| | 100 nM | 10 nM | 5 nM | 0.5 nM |
| N-ER-FY013062M2L96 | 79.57 | 69.31 | 78.21 | 63.78 |
| N-ER-FY013062M6L96 | 86.90 | 79.56 | 87.62 | 77.48 |

As can be seen from Table 10, the siRNA conjugate N-ER-FY013062M6L96 provided in the present disclosure has a superior inhibition effect on the ANGPTL3 gene compared with N-ER-FY013062M2L96.

5.2 *In vivo* stability testing in cynomolgus monkeys

9 healthy cynomolgus monkeys were selected for the experiment, with three monkeys per group, including two administration groups and one blank control group. Subcutaneous administration was performed with a single dose of 9 mpk of N-ER-FY013062M6L96 and N-ER-FY013062M2L96. About 1 mL of whole blood was collected from the saphenous vein or cephalic vein at 0.5 h, 1 h, 2 h, 12 h, and 24 h after administration to analyze the parent drug metabolism in plasma. Parent drug percentage (%) = parent drug content (ng/mL)/total drug content (ng/mL) × 100%. The results are shown in Table 11.

Table 11. Metabolic process of parent drug in cynomolgus monkey plasma

| Analysis of metabolic process of the parent drug in cynomolgus monkeys' plasma | | | | | |
|---|---|---|---|---|---|
| Sample name | Parent drug percentage (%) | | | | |
| | 0.5 h | 1h | 2 h | 12 h | 24 h |
| Antisense strand of N-ER-FY013062M6L96 | 83 | 82 | 82 | 74 | Less than LLOQ |
| Antisense strand of N-ER-FY013062M2L96 | 64 | 55 | 50 | 46 | Less than LLOQ |

As can be seen from Table 11, the proportion of the antisense strand content of N-ER-FY013062M6L96 in plasma was significantly higher than that of N-ER-FY013062M2L96 at each time point, indicating that the stability of N-ER-FY013062M6L96 is better than that of N-ER-FY013062M2L96.

Example 6: Stability Study of N-ER-FY013062M2L96 Antisense Strand and N-ER-FY013062M6L96 Antisense Strand

6.1 Sample preparation

Samples of the N-ER-FY013062M2L96 antisense strand (Table 12, SEQ ID NO: 29) (in an aqueous solution at a concentration of about 10 mg/mL, theoretical molecular weight: 6938.59) and the N-ER-FY013062M6L96 antisense strand (Table 12, SEQ ID NO: 15) (in an aqueous solution at a concentration of about 10 mg/mL, theoretical molecular weight: 6858.61) were stored at room temperature for 72 h and 120 h. At each time point, 10 μL of the sample of the N-ER-FY013062M2L96 antisense strand and 10 μL of the sample of the N-ER-FY013062M6L96 antisense strand were transferred into 600 μL EP tubes, diluted, and mixed uniformly. The concentration of each diluted sample was about 3 mg/mL.

Table 12

| siRNA ID | Antisense strand (5'->3') | SEQ ID NO: |
|---|---|---|
| N-ER-FY013062M2L96 antisense strand | P1mUsUfsmAmUmAGfmAmGmUmAmUmAmACfmCUfmUmCmCsTsT | 29 |
| N-ER-FY013062M6L96 antisense strand | mUsUfsmAmUmAGfmAmGmUmAmUmAmACfmCUfmUmCmCsTsT | 15 |

6.2 Analysis and determination

6.2.1 Mobile phase preparation:

Mobile phase A: 1.5 mL of hexafluoroisopropanol, 70 μL of N,N-diisopropylethylamine, and 200 mL of ultrapure water were added to a mobile phase flask. The mixture was mixed uniformly, shaken, and sonicated. The product was labeled as mobile phase A.
Mobile phase B: 1.5 mL of hexafluoroisopropanol, 70 μL of N,N-diisopropylethylamine, and 200 mL of methanol were added to a mobile phase flask, and the mixture was mixed uniformly, shaken, and sonicated. The product was labeled as mobile phase B.

6.2.2 Chromatographic parameters:

Chromatographic column: ACQUITY Premier Oligonucleotide BEH C18, 2.1 × 150 mm, 1.7 μm
Flow rate: 0.3 mL/min
Sample injection volume: 1 μL
Column oven: 60 °C
Detection wavelength: 260 nm
Injector temperature: 15 °C

The elution gradient is shown in Table 13.

Table 13

| Time | A% | B% |
|---|---|---|
| 0 | 85 | 15 |
| 5 | 40 | 60 |
| 5.1 | 85 | 15 |
| 6 | 85 | 15 |

6.2.3 Mass spectrometry parameters

Mode: Full scan
Mass range: High (400-5000 m/z)
Polarity: Negative
Scan rate: 1 Hz

Cone voltage: Default (40 V)
Capillary voltage: Default (0.8 kV)
Desolvation temperature: Default (550 °C)

6.2.4 Data processing

The m/z responses of the theoretical molecular weights were extracted from the TIC chromatograms of the sample N-ER-FY013062M2-AS and the sample N-ER-FY013062M6-AS, respectively, and compared with those of the 0 h sample at each time point. The results are shown in Table 14.

Table 14

| Sample name | Sample change rate (%) | |
|---|---|---|
| | 72 h | 120 h |
| N-ER-FY013062M2L96 antisense strand | -4.78 | -26.17 |
| N-ER-FY013062M6L96 antisense strand | 4.47 | 2.07 |

As can be seen from Table 14, the single-stranded solution of the N-ER-FY013062M6L96 antisense strand showed no significant change, with a change rate of no more than 5% at each time point within 120 h. The single-stranded solution of the N-ER-FY013062M2L96 antisense strand showed a change rate of - 26.17% after being stored at room temperature for 120 h. The stability of the single-stranded solution of the N-ER-FY013062M6L96 antisense strand is superior to that of the single-stranded solution of the N-ER-FY013062M2L96 antisense strand.

Example 7: Repeated Dose Toxicity Test of N-ER-FY013062M6L96 in SD Rats via Subcutaneous Injection

In this test, 4 groups were set. In the main test group, each group consisted of 15 female and 15 male SD rats. The rats were subcutaneously injected with vehicle control (0.9% sodium chloride injection) or N-ER-FY013062M6L96 at 30 mg/kg, 100 mg/kg, or 300 mg/kg. Administration was performed once every two weeks for a total of 3 administrations, at a dose volume of 5 mL/kg. Scheduled necropsies were performed on 10 female and 10 male animals per group at the end of the administration period on D30, and on the remaining animals in the recovery period on D84. Satellite groups (with the same administration doses and administration route as the main test groups) were set, with 5 female and 5 male rats per group for blood sampling for cytokine analysis. In addition, TK groups (with the same administration route and doses as the main test groups) were set: 4 female and 4 male rats in the vehicle control group, and 8 female and 8 male rats in each test article group for blood sampling for TK analysis.

Toxicity parameters in this test included: mortality/moribundity, general observations, body weight, food consumption, ophthalmic examination, clinical pathology parameters (hematology and coagulation parameters, serum biochemistry parameters, immune function parameters, urinalysis parameters, bone marrow smears), gross necropsy, organ weight, histopathological examination, and tissue distribution. Blood samples of the animals in the satellite groups were collected for cytokine analysis during the adaptation period and 4 h and 24 h after the administration on D1 and D29. Blood samples of the animals in the TK groups were collected before the administration and 0.25 h, 0.5 h, 1 h, 2 h, 4 h, 6 h, 8 h, and 24 h after the administration on D1 and D29 for toxicokinetic analysis.

During the test, all animals survived to the scheduled necropsy. No test article-related obvious abnormalities were observed in general observations, body weight, food consumption, ophthalmic examination, cytokines, clinical pathology parameters (hematology, coagulation, serum biochemistry, immune function parameters, urinalysis parameters, bone marrow smears), or gross necropsy in any animals. Therefore, the no observed adverse effect level (NOAEL) for subcutaneous injection of N-ER-FY013062M6L96 in SD rats was determined.

Example 8: Four-Week Repeated Dose Toxicity Test with Eight-Week Recovery Period of N-ER-FY013062M6L96 in Cynomolgus Monkeys via Subcutaneous Injection

In this study, 4 groups were set, i.e., a vehicle control group (0.9% sodium chloride injection) and N-ER-FY013062M6L96 low-dose (30 mg/kg), mid-dose (100 mg/kg) and high-dose (300 mg/kg) groups, with a dose volume of 2 mL/kg for all groups. Administration was performed once every two weeks for a total of 3 consecutive doses, followed by an eight-week recovery period. Each group comprised 5 animals per sex for the observation of toxicological parameters.

Toxicity parameters observed in this test included: mortality/moribundity, general observations, subcutaneous irritation at the injection site, body weight, food consumption, ophthalmic examination, body temperature, electrocardiogram and blood pressure measurements, as well as examinations of cardiovascular, respiratory and central nervous

system parameters, and clinical pathology parameters (hematology, coagulation, serum biochemistry, and urinalysis parameters) and immunology parameters (immunocyte phenotyping, complement, immunoglobulin, and cytokine parameters). Necropsies were performed at the end of the administration period (D30) and at the end of the recovery period (D85). 3 animals per sex per group were necropsied at the end of the administration period, and 2 animals per sex per group were necropsied at the end of the recovery period. All necropsied animals were subjected to gross pathological examination, organ weighing, bone marrow smears, and histopathological examination. Meanwhile, blood samples were collected from the animals before administration and 0.25 h, 0.5 h, 1 h, 2 h, 4 h, 8 h, and 24 h after administration on D1-D2 and D29-D30 for toxicokinetic (TK) studies. Blood samples were collected after grouping, on D14, D28, D56, and D84 for anti-drug antibody analysis. In addition, the livers and kidneys were collected from scheduled necropsy animals on the day of necropsy for the tissue distribution study.

During the experiment, all animals survived to the scheduled necropsy. No abnormal changes related to the administration of N-ER-FY013062M6L96 were noted in clinical observations, body weight, food consumption, body temperature, ophthalmic examination, electrocardiogram, hematology and coagulation function parameters, urinalysis parameters, complement parameters, immunoglobulin parameters, cytokines, bone marrow smears, or gross necropsy.

Example 9: Efficacy Experiment of siRNA Conjugates in Cynomolgus Monkeys

6 cynomolgus monkeys (weighing 2-4 kg) aged 3-6 years (SAFE Medical Technology Co., Ltd.) were randomly divided into 2 groups and subjected to 14 days of adaptive feeding before administration. The day of administration was defined as Day 1 (D1). The specific administration design is summarized in Table 15.

Table 15

| Compound | Route of administration | Dose (mg/kg) | Concentration (mg/mL) | Administration volume (mL/kg) |
|---|---|---|---|---|
| Negative control (1× PBS buffer) | Subcutaneous administration | - | - | 0.5 |
| N-ER-FY013062M6L96 | | 9 | 18 | 0.5 |

Injection sites were the dorsal lumbar skin or other appropriate sites. The hair around the injection site was shaved clean, the skin was disinfected with iodine and alcohol, and then subcutaneous administration was performed. Blood samples were collected on D1 (before administration) and on D8, D15, D22, D29, D36, D43, D50, D57, D64, D71, D78, and D84 after administration for ANGPTL3 protein expression levels and triglyceride (TG) assay. The results are shown in Tables 16 and 17.

Table 16

| Group | ANGPTL3 protein rate of change in plasma (%) | | | | | | | | | | | | |
|---|---|---|---|---|---|---|---|---|---|---|---|---|---|
| | D1 | D8 | D15 | D22 | D29 | D36 | D43 | D50 | D57 | D64 | D71 | D78 | D84 |
| Negative control (1× PBS buffer) | 0 | -7 | 14 | -7 | -7 | -12 | -12 | -8 | -1 | -8 | -11 | -12 | -11 |
| N-ER-FY013062M6L96 | 0 | -76 | -82 | -87 | -86 | -85 | -85 | -79 | -75 | -75 | -67 | -67 | -66 |

Table 17

| Group | TG rate of change in plasma (%) | | | | | | | | | | | | |
|---|---|---|---|---|---|---|---|---|---|---|---|---|---|
| | D1 | D8 | D15 | D22 | D29 | D36 | D43 | D50 | D57 | D64 | D71 | D78 | D84 |
| Negative control (1× PBS buffer) | 0 | -13 | -12 | -2 | -23 | -12 | -1 | -15 | -28 | 14 | 19 | 10 | 29 |
| N-ER-FY013062M6L96 | 0 | -71 | -78 | -79 | -81 | -77 | -75 | -75 | -75 | -68 | -63 | -62 | -54 |

The siRNA conjugate N-ER-FY013062M6L96 of the present disclosure can significantly reduce the expression level of the ANGPTL3 protein and triglyceride content in plasma of the cynomolgus monkeys.

The examples described above of the present disclosure are provided merely as illustrative examples for clearly

explaining the present disclosure, and are not intended to limit the implementations of the present disclosure. Various changes or modifications can be made by those of ordinary skill in the art on the basis of the above description. It is unnecessary and impossible to exhaustively list all the embodiments herein. Any modifications, equivalent substitutions, improvements, and the like made within the spirit and principles of the present disclosure shall fall within the protection scope of the claims of the present disclosure.

**Claims**

1. A modified siRNA for inhibiting ANGPTL3 gene expression, comprising a sense strand and an antisense strand, wherein each nucleotide in the modified siRNA is independently a modified or unmodified nucleotide; the sense strand comprises a nucleotide sequence I, the antisense strand comprises a nucleotide sequence II, and the nucleotide sequence I is at least partially reversely complementary to the nucleotide sequence II to form a double-stranded region, wherein the nucleotide sequence I and the nucleotide sequence II are selected from the following sequences:

 (1) nucleotide sequence I comprising the nucleotide sequence as shown in SEQ ID NO: 35, and nucleotide sequence II comprising the nucleotide sequence as shown in SEQ ID NO: 36:

 5'-GGAUUAUCUUGGAAGUCUA-3' (SEQ ID NO: 35)
 5'-UAGACUUCCAAGAUAAUCC-3' (SEQ ID NO: 36);

 (2) nucleotide sequence I comprising the nucleotide sequence as shown in SEQ ID NO: 37, and nucleotide sequence II comprising the nucleotide sequence as shown in SEQ ID NO: 38:

 5'-GUCUCAAAAUGGAAGGUU$Z_1$-3' (SEQ ID NO: 37)
 5'-$Z_2$AACCUUCCAUUUUGAGAC-3' (SEQ ID NO: 38)
 wherein $Z_1$ is A, and $Z_2$ is U; or $Z_1$ is U, and $Z_2$ is A;

 (3) nucleotide sequence I comprising the nucleotide sequence as shown in SEQ ID NO: 27, and nucleotide sequence II comprising the nucleotide sequence as shown in SEQ ID NO: 28:

 5'-GGAAGGUUAUACUCUAUA$Z_3$-3' (SEQ ID NO: 27)
 5'-$Z_4$UAUAGAGUAUAACCUUCC-3' (SEQ ID NO: 28)
 wherein $Z_3$ is A, and $Z_4$ is U; or $Z_3$ is U, and $Z_4$ is A.

2. The modified siRNA according to claim 1, wherein the nucleotide sequence I is substantially reversely complementary, virtually reversely complementary, or completely reversely complementary to the nucleotide sequence II; the substantially reversely complementary means that there are no more than 3 base mismatches between the two nucleotide sequences; the virtually reversely complementary means that there is no more than 1 base mismatch between the two nucleotide sequences; the completely reversely complementary means that there is no base mismatch between the two nucleotide sequences.

3. The modified siRNA according to claim 1 or 2, wherein the sense strand further comprises a nucleotide sequence III, and the antisense strand further comprises a nucleotide sequence IV, wherein the nucleotide sequence III and the nucleotide sequence IV have a length of 0-3 nucleotides; the nucleotide sequence III is linked to the 3' end of the sense strand to form a 3' overhang of the sense strand, and/or the nucleotide sequence IV is linked to the 3' end of the antisense strand to form a 3' overhang of the antisense strand; preferably, the nucleotide sequence III or the nucleotide sequence IV has a length of 0-2 nucleotides; more preferably, the nucleotide sequence III or the nucleotide sequence IV is two thymine deoxyribonucleotides.

4. The modified siRNA according to any one of claims 1-3, wherein the double-stranded region has a length of 15-30 nucleotide pairs; preferably, the double-stranded region has a length of 17-23 nucleotide pairs; more preferably, the double-stranded region has a length of 19-23 nucleotide pairs.

5. The modified siRNA according to any one of claims 1-4, wherein the sense strand or the antisense strand has 15-30 nucleotides; preferably, the sense strand or the antisense strand has 19-25 nucleotides; more preferably, the sense strand or the antisense strand has 19-23 nucleotides.

6. The modified siRNA according to any one of claims 1-5, wherein at least one nucleotide in the sense strand or the antisense strand is a modified nucleotide, and/or at least one phosphoester group is a phosphoester group with a modification group; preferably, the phosphoester group with a modification group is a phosphorothioate diester bond formed by substituting one oxygen atom in a phosphodiester bond in the phosphoester group with a sulfur atom; and/or, the siRNA comprises a sense strand without a 3' overhang nucleotide.

7. The modified siRNA according to any one of claims 1-6, wherein the 5' end nucleotide of the antisense strand is not linked to a 5' phosphate group or a 5' phosphate-derived group.

8. The modified siRNA according to any one of claims 1-7, wherein the modified nucleotide is selected from a 2'-fluoro-modified nucleotide, a 2'-alkoxy-modified nucleotide, a 2'-substituted alkoxy-modified nucleotide, a 2'-alkyl-modified nucleotide, a 2'-substituted alkyl-modified nucleotide, a 2'-deoxynucleotide, a 2'-amino-modified nucleotide, a 2'-substituted amino-modified nucleotide, a nucleotide analog, and a combination of any two or more thereof; preferably, the modified nucleotide is selected from a 2'-fluoro-modified nucleotide, a 2'-methoxy-modified nucleotide, a 2'-O-$CH_2$-$CH_2$-O-$CH_3$-modified nucleotide, a 2'-O-$CH_2$-CH=$CH_2$-modified nucleotide, a 2'-$CH_2$-$CH_2$-CH=$CH_2$-modified nucleotide, a 2'-deoxynucleotide, a nucleotide analog, and a combination of any two or more thereof;
the nucleotide analog is selected from an isonucleotide, LNA, ENA, cET, UNA, and GNA.

9. The modified siRNA according to any one of claims 1-8, wherein in the direction from the 5' end to the 3' end, ribonucleotides at positions 7, 9, 10, and 11 in the sense strand are 2'-F-modified ribonucleotides, and ribonucleotides at remaining positions in the sense strand are 2'-O-$CH_3$-modified ribonucleotides.

10. The modified siRNA according to any one of claims 1-9, wherein the sense strand comprises phosphorothioate diester bonds located at the positions shown below:

a position between the first nucleotide and the second nucleotide from the 5' end of the sense strand; and
a position between the second nucleotide and the third nucleotide from the 5' end of the sense strand;
or, the sense strand comprises phosphorothioate diester bonds located at the positions shown below:

a position between the first nucleotide and the second nucleotide from the 5' end of the sense strand;
a position between the second nucleotide and the third nucleotide from the 5' end of the sense strand;
a position between the first nucleotide and the second nucleotide from the 3' end of the sense strand; and
a position between the second nucleotide and the third nucleotide from the 3' end of the sense strand.

11. The modified siRNA according to any one of claims 1-10, wherein in the direction from the 5' end to the 3' end, ribonucleotides at positions 2, 6, 14, and 16 in the antisense strand are 2'-F-modified ribonucleotides, and ribonucleotides at remaining positions in the antisense strand are 2'-O-$CH_3$-modified ribonucleotides;

or, in the direction from the 5' end to the 3' end, ribonucleotides at positions 2, 6, 8, 9, 14, and 16 in the antisense strand are 2'-F-modified ribonucleotides, and ribonucleotides at remaining positions in the antisense strand are 2'-O-$CH_3$-modified ribonucleotides;
or, in the direction from the 5' end to the 3' end, ribonucleotides at positions 2, 14, and 16 in the antisense strand are 2'-F-modified ribonucleotides, a ribonucleotide at position 6 in the antisense strand is a ribonucleotide modified with a nucleotide derivative GNA, and ribonucleotides at remaining positions in the antisense strand are 2'-O-$CH_3$-modified ribonucleotides;
or, in the direction from the 5' end to the 3' end, ribonucleotides at positions 2, 6, 14, and 16 in the antisense strand are 2'-F-modified ribonucleotides, a ribonucleotide at position 7 in the antisense strand is a ribonucleotide modified with a nucleotide derivative GNA, and the ribonucleotides at remaining positions in the antisense strand are 2'-O-$CH_3$-modified ribonucleotides.

12. The modified siRNA according to any one of claims 1-11, wherein the antisense strand comprises phosphorothioate diester bonds located at the positions shown below:

a position between the first nucleotide and the second nucleotide from the 5' end of the antisense strand;
a position between the second nucleotide and the third nucleotide from the 5' end of the antisense strand;
a position between the first nucleotide and the second nucleotide from the 3' end of the antisense strand; and
a position between the second nucleotide and the third nucleotide from the 3' end of the antisense strand.

13. The modified siRNA according to any one of claims 1-12, wherein the modified siRNA comprises or is selected from the modified siRNAs in Table 1; preferably, the modified siRNA is N-ER-FY013062M6 or N-ER-FY013062M6D2.

14. A siRNA conjugate, comprising the modified siRNA according to any one of claims 1-13, and a conjugated group conjugated to the modified siRNA.

15. The siRNA conjugate according to claim 14, wherein the conjugated group has the structure shown below:

formula (I)

formula (III)

formula (IV)

formula (V)

formula (VI)

formula (VII)

formula (VIII)

formula (IX).

16. The siRNA conjugate according to claim 14 or 15, wherein the conjugated group is linked to the 3' end of the sense strand.

17. The siRNA conjugate according to claim 16, wherein the conjugated group is conjugated to the 3' end of the sense strand via a phosphodiester bond;
preferably, the sense strand and the antisense strand of the siRNA conjugate are complementary to form a double-stranded region of the siRNA conjugate, the 3' end of the sense strand forms a blunt end, and the 3' end of the

antisense strand has 1-2 overhanging nucleotides extending out of the double-stranded region.

**18.** The siRNA conjugate according to any one of claims 14-17, wherein the siRNA conjugate has the structure shown below:

(formula II),

wherein the double helical structure is a modified siRNA.

**19.** The siRNA conjugate according to any one of claims 14-18, wherein the siRNA conjugate is formed by linking any one of the modified siRNAs shown in Table 1 to a conjugated group;
preferably, in the siRNA conjugate, the sense strand and the antisense strand are selected from the combinations of the sense strand and the antisense strand of the siRNA conjugates shown in Table 2; preferably, the siRNA conjugate is N-ER-FY013062M6L96.

**20.** A pharmaceutical composition, comprising at least one of the following: the modified siRNA according to any one of claims 1-13 and the siRNA conjugate according to any one of claims 14-19.

**21.** The pharmaceutical composition according to claim 20, wherein the pharmaceutical composition further comprises one or more pharmaceutically acceptable carriers.

**22.** Use of the modified siRNA according to any one of claims 1-13, the siRNA conjugate according to any one of claims 14-19, or the pharmaceutical composition according to claim 20 or 21 in at least one of the following:

(1) inhibiting ANGPTL3 gene expression, or preparing a medicament for inhibiting ANGPTL3 gene expression;
(2) preventing or treating a disease associated with abnormal ANGPTL3 gene expression, or preparing a medicament for preventing or treating a disease associated with abnormal ANGPTL3 gene expression;
(3) treating a subject suffering from a disease that would benefit from a reduction in ANGPTL3 gene expression, or preparing a medicament for treating a subject suffering from a disease that would benefit from a reduction in ANGPTL3 gene expression.

**23.** The use according to claim 22, wherein the disease associated with abnormal ANGPTL3 gene expression is selected from a disease associated with lipid metabolism;
optionally, the disease associated with lipid metabolism is selected from the group consisting of the following diseases:
chylomicronemia syndrome, type 2 diabetes, familial partial lipodystrophy, hypercholesterolemia, familial hypercholesterolemia, non-alcoholic fatty liver, atherosclerosis, hypertriglyceridemia, knee injury and osteoarthritis, dyslipidemia (including mixed dyslipidemia), and fatty liver.

**24.** A method for inhibiting intracellular ANGPTL3 gene expression *in vivo* or *in vitro*, comprising contacting a cell expressing ANGPTL3 with, or administering to a subject in need thereof, a therapeutically effective amount of the modified siRNA according to any one of claims 1-13, the siRNA conjugate according to any one of claims 14-19, or the pharmaceutical composition according to claim 20 or 21.

**25.** A method for preventing or treating a disease associated with abnormal ANGPTL3 gene expression, comprising administering to a subject in need thereof a therapeutically effective amount of the modified siRNA according to any one of claims 1-13, the siRNA conjugate according to any one of claims 14-19, or the pharmaceutical composition according to claim 20 or 21,

wherein preferably, the disease associated with abnormal ANGPTL3 gene expression is selected from a disease associated with lipid metabolism;
optionally, the disease associated with lipid metabolism is selected from the group consisting of the following diseases:
chylomicronemia syndrome, type 2 diabetes, familial partial lipodystrophy, hypercholesterolemia, familial hypercholesterolemia, non-alcoholic fatty liver, atherosclerosis, hypertriglyceridemia, knee injury and osteoarthritis, dyslipidemia (including mixed dyslipidemia), and fatty liver.

**26.** The modified siRNA according to any one of claims 1-13, the siRNA conjugate according to any one of claims 14-19, or the pharmaceutical composition according to claim 20 or 21 for use in therapy.

**INTERNATIONAL SEARCH REPORT**

International application No.
**PCT/CN2024/132007**

**A. CLASSIFICATION OF SUBJECT MATTER**

C12N15/113(2010.01)i； A61P19/08(2006.01)i； A61P3/00(2006.01)i； A61K31/713(2006.01)i； A61K47/54(2017.01)i； A61P3/06(2006.01)i

According to International Patent Classification (IPC) or to both national classification and IPC

**B. FIELDS SEARCHED**

Minimum documentation searched (classification system followed by classification symbols)

IPC:C12N A61P A61K

Documentation searched other than minimum documentation to the extent that such documents are included in the fields searched

Electronic data base consulted during the international search (name of data base and, where practicable, search terms used)

CNTXT; WPABS; ENTXT; USTXT; JPTXT; KRTXT; DWPI; VEN; CJFD; ISI web of Science; PUBMED; 百度学术, Baidu Scholar; 百度, Baidu; 读秀, Duxiu; 万方, Wanfang; CNKI; Genbank; EBI; ENA; STN: 血管生成素-样蛋白3, siRNA, 干扰RNA, ANGPTL3, ANGPL3, ANG3, 修饰, 缀合, 偶联, 脂代谢, 血脂异常, GalNAc, angiopoietin like 3, conjugate, dyslipidemia, modified, SEQ ID NOs: 1-38的序列, SEQ ID NOs: 1-38

**C. DOCUMENTS CONSIDERED TO BE RELEVANT**

| Category* | Citation of document, with indication, where appropriate, of the relevant passages | Relevant to claim No. |
|---|---|---|
| X | CN 116987696 A (BEIJING FOYOU PHARMACEUTICAL CO., LTD.) 03 November 2023 (2023-11-03)<br>claims 1-45, and description, paragraphs 0654-0677 | 1-26 |
| X | CN 115572725 A (SHANGHAI JUNSHI BIOSCIENCES CO., LTD. et al.) 06 January 2023 (2023-01-06)<br>claims 1-27 | 1-26 |
| X | CN 116887842 A (SANOFI SA) 13 October 2023 (2023-10-13)<br>claims 1-40, and description, paragraphs 0095, 0119-0121 and 0125-0126 | 1-26 |
| X | US 2023257750 A1 (NANOPEPTIDE (QINGDAO) BIOTECHNOLOGY LTD.) 17 August 2023 (2023-08-17)<br>claims 1-14, and description, paragraphs 0094-0118 | 1-26 |
| X | EP 4092120 A1 (ALNYLAM PHARMACEUTICALS, INC.) 23 November 2022 (2022-11-23)<br>claims 1-19, and description, paragraphs 0141-0158 and 0364 | 1-26 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 February 2025** | **19 February 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2024/132007**

**C. DOCUMENTS CONSIDERED TO BE RELEVANT**

| Category* | Citation of document, with indication, where appropriate, of the relevant passages | Relevant to claim No. |
|---|---|---|
| X | CN 116888263 A (SHANGHAI ARGO BIOPHARMACEUTICAL CO., LTD.) 13 October 2023 (2023-10-13) claims 1-93, and description, paragraph 055 | 1-26 |
| A | CN 111973617 A (SUZHOU RIBO LIFE SCIENCE CO., LTD.) 24 November 2020 (2020-11-24) claims 1-10 | 1-26 |

Form PCT/ISA/210 (second sheet) (July 2022)

**INTERNATIONAL SEARCH REPORT**

International application No.
**PCT/CN2024/132007**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **22-25**
because they relate to subject matter not required to be searched by this Authority, namely:

Claims 22-25 relate to a method for treating a disease, which falls within methods for treatment of diseases as defined in PCT Rule 39.1(iv). However, a search is still performed on the basis of the pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/132007**

| Patent document cited in search report | Publication date (day/month/year) | Patent family member(s) | Publication date (day/month/year) |
|---|---|---|---|
| CN 116987696 A | 03 November 2023 | WO 2024027518 A1 | 08 February 2024 |
| CN 115572725 A | 06 January 2023 | CA 3224904 A1 | 29 December 2022 |
| | | WO 2022268054 A1 | 29 December 2022 |
| | | JP 2024523458 A | 28 June 2024 |
| | | AU 2022298603 A1 | 01 February 2024 |
| | | MX 2024000237 A | 15 March 2024 |
| | | KR 20240023635 A | 22 February 2024 |
| | | EP 4361266 A1 | 01 May 2024 |
| | | BR 112023026971 A2 | 12 March 2024 |
| CN 116887842 A | 13 October 2023 | WO 2022079222 A1 | 21 April 2022 |
| | | EP 4229200 A1 | 23 August 2023 |
| | | US 2023383294 A1 | 30 November 2023 |
| | | JP 2023546103 A | 01 November 2023 |
| US 2023257750 A1 | 17 August 2023 | US 2024067971 A1 | 29 February 2024 |
| | | JP 2023121159 A | 30 August 2023 |
| | | JP 7588887 B2 | 25 November 2024 |
| | | EP 4331608 A1 | 06 March 2024 |
| | | EP 4223875 A1 | 09 August 2023 |
| | | WO 2022068923 A1 | 07 April 2022 |
| | | WO 2022068923 A9 | 13 October 2022 |
| | | JP 2023536685 A | 29 August 2023 |
| | | JP 7607957 B2 | 06 January 2025 |
| EP 4092120 A1 | 23 November 2022 | SG 10201913683 WA | 30 March 2020 |
| | | NZ 620151 A | 29 April 2016 |
| | | CA 2839573 A1 | 27 December 2012 |
| | | KR 20230107908 A | 18 July 2023 |
| | | US 2022073925 A1 | 10 March 2022 |
| | | US 11306315 B2 | 19 April 2022 |
| | | EP 3656860 A1 | 27 May 2020 |
| | | EP 3656860 B1 | 20 April 2022 |
| | | MX 2022012087 A | 30 November 2022 |
| | | SG 10201800715 PA | 27 February 2018 |
| | | US 2019352645 A1 | 21 November 2019 |
| | | US 10550390 B2 | 04 February 2020 |
| | | US 2020140866 A1 | 07 May 2020 |
| | | US 10934545 B2 | 02 March 2021 |
| | | EP 3444348 A1 | 20 February 2019 |
| | | AU 2022201993 A1 | 14 April 2022 |
| | | AU 2022201993 B2 | 29 August 2024 |
| | | ES 2923573 T3 | 28 September 2022 |
| | | KR 20140044870 A | 15 April 2014 |
| | | KR 102232287 B1 | 29 March 2021 |
| | | US 2019316137 A1 | 17 October 2019 |
| | | US 10557139 B2 | 11 February 2020 |
| | | US 2022073927 A1 | 10 March 2022 |
| | | US 11866709 B2 | 09 January 2024 |
| | | JP 2023052363 A | 11 April 2023 |
| | | MX 360782 B | 16 November 2018 |
| | | BR 122019026068 B1 | 20 September 2022 |
| | | BR 122019026068 B8 | 18 October 2022 |

Form PCT/ISA/210 (patent family annex) (July 2022)

**INTERNATIONAL SEARCH REPORT**
**Information on patent family members**

International application No.
**PCT/CN2024/132007**

| Patent document cited in search report | Publication date (day/month/year) | Patent family member(s) | | | Publication date (day/month/year) |
|---|---|---|---|---|---|
| | | US | 2024175030 | A1 | 30 May 2024 |
| | | US | 2022073928 | A1 | 10 March 2022 |
| | | US | 11840692 | B2 | 12 December 2023 |
| | | US | 2017355994 | A1 | 14 December 2017 |
| | | US | 10337010 | B2 | 02 July 2019 |
| | | US | 2021171954 | A1 | 10 June 2021 |
| | | US | 11332743 | B2 | 17 May 2022 |
| | | US | 2022073929 | A1 | 10 March 2022 |
| | | US | 11834662 | B2 | 05 December 2023 |
| | | US | 2022073926 | A1 | 10 March 2022 |
| | | US | 11306316 | B2 | 19 April 2022 |
| | | US | 2022235358 | A1 | 28 July 2022 |
| | | US | 11525138 | B2 | 13 December 2022 |
| | | WO | 2012177784 | A2 | 27 December 2012 |
| | | WO | 2012177784 | A3 | 21 February 2013 |
| | | US | 2022073924 | A1 | 10 March 2022 |
| | | US | 11306314 | B2 | 19 April 2022 |
| | | US | 2016186180 | A1 | 30 June 2016 |
| | | US | 9771591 | B2 | 26 September 2017 |
| | | MX | 2013014364 | A | 30 July 2014 |
| | | MX | 345095 | B | 17 January 2017 |
| | | KR | 20220063292 | A | 17 May 2022 |
| | | KR | 102554896 | B1 | 12 July 2023 |
| | | RU | 2019138721 | A | 26 April 2021 |
| | | RU | 2019138721 | A3 | 02 December 2021 |
| | | AU | 2012272970 | A1 | 06 February 2014 |
| | | AU | 2012272970 | A8 | 27 February 2014 |
| | | EP | 2723758 | A2 | 30 April 2014 |
| | | EP | 2723758 | A4 | 15 April 2015 |
| | | EP | 2723758 | B1 | 20 June 2018 |
| | | RU | 2014101627 | A | 27 July 2015 |
| | | RU | 2711799 | C2 | 22 January 2020 |
| | | AU | 2019226296 | A1 | 26 September 2019 |
| | | US | 2014179768 | A1 | 26 June 2014 |
| | | US | 9322018 | B2 | 26 April 2016 |
| | | KR | 20210035317 | A | 31 March 2021 |
| | | KR | 102395085 | B1 | 09 May 2022 |
| | | BR | 112013033260 | A2 | 22 November 2016 |
| | | BR | 112013033260 | A8 | 19 December 2017 |
| | | BR | 112013033260 | B1 | 21 June 2022 |
| | | JP | 2014524738 | A | 25 September 2014 |
| | | JP | 6110372 | B2 | 05 April 2017 |
| | | MX | 2023000053 | A | 10 April 2023 |
| | | AU | 2024266805 | A1 | 12 December 2024 |
| | | US | 2021171955 | A1 | 10 June 2021 |
| | | US | 11130953 | B2 | 28 September 2021 |
| | | JP | 2017148048 | A | 31 August 2017 |
| | | JP | 6430559 | B2 | 28 November 2018 |
| | | JP | 2019013252 | A | 31 January 2019 |
| | | JP | 6698787 | B2 | 27 May 2020 |

Form PCT/ISA/210 (patent family annex) (July 2022)

**INTERNATIONAL SEARCH REPORT**
**Information on patent family members**

International application No.
**PCT/CN2024/132007**

| Patent document cited in search report | Publication date (day/month/year) | Patent family member(s) | Publication date (day/month/year) |
|---|---|---|---|
| | | AU 2021201547 A1 | 01 April 2021 |
| | | AU 2021201547 B2 | 07 April 2022 |
| | | JP 2020174671 A | 29 October 2020 |
| | | JP 7245194 B2 | 23 March 2023 |
| | | HK 1199457 A1 | 03 July 2015 |
| | | AU 2017206228 A1 | 10 August 2017 |
| CN 116888263 A | 13 October 2023 | TW 202321452 A | 01 June 2023 |
| | | JP 2024536838 A | 08 October 2024 |
| | | KR 20240053627 A | 24 April 2024 |
| | | AU 2022352799 A1 | 21 March 2024 |
| | | IL 310929 A | 01 April 2024 |
| | | US 2024376475 A1 | 14 November 2024 |
| | | CA 3230527 A1 | 30 March 2023 |
| | | WO 2023045994 A1 | 30 March 2023 |
| | | MX 2024003592 A | 10 April 2024 |
| | | EP 4405476 A1 | 31 July 2024 |
| CN 111973617 A | 24 November 2020 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SHARP et al.** *Genes Dev.*, 2001, vol. 15, 485
- **BERNSTEIN et al.** *Nature*, 2001, vol. 409, 363
- **NYKANEN et al.** *Cell*, 2001, vol. 107, 309
- **ELBASHIR et al.** *Genes Dev.*, 2001, vol. 15, 188
- **MINICOCCI et al.** Mutations in the ANGPTL3 gene and familial combined hypolipidemia: a clinical and biochemical characterization.. *J Clin Endocrinol Metab*, 2012, vol. 97 (7), E1266-1275
- **KAPLAN et al.** Regulation of the angiopoietin-like protein 3 gene by LXR.. *J Lipid Res*, 2003, vol. 44 (1), 136-143
- **CHENG HAIPENG et al.** Lipoprotein lipase degradation and metabolic disorders. *Chinese Journal of Arteriosclerosis*, 2017, vol. 25 (05), 513-518
- **RAO JIA et al.** Expression of ANGPTL3 in children with primary nephrotic syndrome. *Chinese Journal of Nephrology*, 2006, vol. 22 (05), 286-290
- **STITZIEL et al.** ANGPTL3 deficiency and protection against coronary artery disease.. *J Am Coll Cardiol*, 2017, vol. 69 (16), 2054-2063
- **WANG PF et al.** Clinical significance of angiopoietin-like protein 3 expression in patients with glioblastoma.. *Neoplasma*, 2016, vol. 63 (1), 93-98
- **MUTHIAH MANOHARAN**. siRNA conjugates carrying sequentially assembled trivalent N-acetylgalactosamine linked through nucleosides elicit robust gene silencing in vivo in hepatocytes.. *ACS Chemical biology*, 2015, vol. 10 (5), 1181-7
- **SAMBROOK et al.** Molecular Cloning: A Laboratory Manual. Cold Spring Harbor Laboratory Press, 1989